(19)

Europäisches Patentamt
European Patent Office
Office européen des brevets

(11) **EP 2 065 721 A2**

(12) **EUROPEAN PATENT APPLICATION**

(43) Date of publication:
**03.06.2009 Bulletin 2009/23**

(51) Int Cl.:
*G01S 5/02* (2006.01)     *G01S 5/00* (2006.01)
*G01S 5/14* (2006.01)     *G01C 21/00* (2006.01)

(21) Application number: **08253468.6**

(22) Date of filing: **24.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **27.11.2007 JP 2007305956**

(71) Applicant: **Sony Corporation**
**Tokyo 108-0075 (JP)**

(72) Inventors:
• **Asukai, Masamichi**
  **Tokyo 108-0075 (JP)**
• **Ito, Taiji**
  **Tokyo 108-0075 (JP)**
• **Sugino, Akinobu**
  **Tokyo 108-0075 (JP)**

• **Sano, Akane**
  **Tokyo 108-0075 (JP)**
• **Hayashi, Kazunori**
  **Tokyo 108-0075 (JP)**
• **Kon, Takayasu**
  **Tokyo 108-0075 (JP)**
• **Kamada, Yasunori**
  **Tokyo 108-0075 (JP)**
• **Takehara, Mitsuru**
  **Tokyo 108-0075 (JP)**
• **Sako, Yoichiro**
  **Tokyo 108-0075 (JP)**
• **Kamatani, Yoshiteru**
  **Tokyo 108-0075 (JP)**

(74) Representative: **Turner, James Arthur et al**
**D Young & Co**
**120 Holborn**
**London EC1N 2DY (GB)**

(54) **Interpersonal relationship evaluation device, interpersonal relationship evaluation method, interpersonal relationship evaluation system, and terminal device**

(57) An interpersonal relationship evaluation method includes the steps of: obtaining position information obtained at each of the two or more terminal devices over a plurality of points in time; and calculating a mean value of distance between the respective terminal devices with a point in time as a target wherein a predetermined condition holds to obtain the mean value as an interpersonal relationship evaluation value regarding each terminal device user.

FIG. 1

EP 2 065 721 A2

**Description**

The present invention relates to an interpersonal relationship evaluation device and a method thereof, and an interpersonal relationship evaluation system and the terminal devices thereof.

There has been available a technique for calculating interpersonal relationship evaluation values for evaluating interpersonal relationship objectively quantitatively based on the behavior history of each user to be observed.

For example, with Japanese Unexamined Patent Application Publication No. 2005-131748, and Japanese Unexamined Patent Application Publication No. 2005-327156, a technique has been disclosed wherein wireless tags carried by users, and a reading device thereof are employed to record the behavior history (movement course) of each user, and evaluate relations between the respective users based on the history information thereof. For example, determination is made based on movement trajectories whether or not a certain user moves on the same course as another user simultaneously, thereby recognizing friendly relationship between the users.

Now, as disclosed in the above-mentioned Japanese Unexamined Patent Application Publication No. 2005-131748, and Japanese Unexamined Patent Application Publication No. 2005-327156 (particularly, Japanese Unexamined Patent Application Publication No. 2005-327156), physical distance between users to be observed has been employed as an index at the time of evaluating interpersonal relationship.

Specifically, with the above-mentioned Japanese Unexamined Patent Application Publication No. 2005-327156, there has been disclosed a technique for obtaining the position information of each user at each point in time, obtaining a mean value of distance between users during a certain period based on the result thereof, and determining whether or not this mean value is equal to or smaller than a predetermined threshold, thereby determining whether or not the users have friendly relationship.

With a technique for obtaining a mean value of distance such as described in Japanese Unexamined Patent Application Publication No. 2005-327156, the longer a period wherein a user to be observed is in the neighborhood is, the better a value is obtained. That is to say, an evaluation value itself is for obtaining "distance", but with this technique, time length wherein a user to be observed is in the neighborhood is regarded as a substantial evaluation index.

However, with such a technique for regarding time length as an evaluation index wherein a user to be observed is in the neighborhood, there is a possibility that, for example, co-workers of a company who are not very intimate have adjacent seats, which causes a misconception wherein they have very friendly relationship, and consequently, interpersonal relationship evaluation is performed incorrectly.

There has been recognized demand to enable interpersonal relationship evaluation to be performed more accurately, thereby preventing occurrence of a misconception regarding interpersonal relationship.

To this end, with an embodiment of the present invention, the following arrangement has been employed as an interpersonal relationship evaluation device for performing interpersonal relationship evaluations based on the information of distance between users.

Specifically, a computing unit is provided, which executes information obtaining processing for obtaining position information obtained over a plurality of points in time at each of two or more terminal devices, and evaluation value calculation processing for calculating a mean value of distance between the respective terminal devices with a point in time as a target wherein a predetermined condition holds, thereby obtaining the mean value thereof as an interpersonal relationship evaluation value regarding each terminal device user.

According to the above-mentioned embodiment of the present invention, a mean value of interpersonal distance is not calculated with all points in time of a period to be evaluated as a target, but calculated with only a point in time as a target wherein a predetermined condition holds.

Thus, a technique has been employed wherein a point in time to be evaluated for calculating a mean value of distance is narrowed down to only a point in time wherein a certain condition is satisfied, whereby an interpersonal relationship evaluation index can be prevented from being dependent on time length wherein a user to be observed is in the neighborhood. Thus, occurrence of a misconception regarding interpersonal relationship can be prevented effectively, such as occurrence of a misconception in the case of employing a technique according to the related art which regards time length wherein a user to be observed is in the neighborhood as an index.

Now, as described above, narrowing down points in time to be evaluated for calculating a mean value of distance to only a point in time wherein a certain condition is satisfied enables a mean value of distance between the respective users to be obtained in a state wherein a predetermined condition is satisfied. Thus, consequently, interpersonal relation can be evaluated based on the behavior (proximity tendency or alienation tendency) of each user under a situation wherein a certain condition is satisfied.

For example, if a condition is set as the above-mentioned predetermined condition wherein the state of each user to be evaluated is a state regarded as particular relationship (e.g., friends or lovers), interpersonal relationship can be evaluated based of the behavior of each user under such a situation regarded as particular relationship.

According to the above-mentioned embodiment of the present invention, points in time to be evaluated for calculating a mean value of interpersonal distance is narrowed down to a point in time wherein a certain condition is satisfied, whereby interpersonal relationship can be evaluated in a more accurate manner without being dependent on only time length wherein each user is in the neighborhood, such as a technique according to the related art.

Various further aspects and features of the invention, such as an interpersonal relationship evaluation system configured of two or more terminal devices and a server device are defined in the appended claims. Combinations of features from the dependent claims may be combined with features of the independent claims as appropriate and not merely as explicitly set out in the claims.

Embodiments of the invention will now be described with reference to the accompanying drawings, throughout which like parts are referred to by like references, and in which:

Fig. 1 is a diagram for describing overview of an interpersonal relationship evaluation system according to an embodiment of the present invention;

Fig. 2 is a block diagram illustrating the internal configuration of a terminal device according to a first embodiment of the present invention;

Fig. 3 is a block diagram illustrating the internal configuration of a server device according an embodiment of the present invention;

Fig. 4 is a diagram for describing accumulation information for distance calculation;

Fig. 5 is a diagram for describing conditions regarding a target range, of conditions to be set for narrowing down points in time to be evaluated for calculating a mean value of distance;

Figs. 6A, 6B and 6C are diagrams for describing conditions regarding placement states, of conditions to be set for narrowing down points in time to be evaluated for calculating a mean value of distance;

Fig. 7 is a diagram for describing an entire flow at the time of calculating interpersonal relationship evaluation values;

Fig. 8 is a diagram schematically illustrating overview of operation to be performed with an interpersonal relationship system according to an embodiment of the present invention;

Figs. 9A,9B, 9C and 9D are diagrams illustrating presentation examples of interpersonal relationship evaluation results;

Fig. 10 is a flowchart illustrating processing operation to be executed at the time of setting a target point in time to be evaluated for calculating a mean value of distance, as processing operation to be executed at a server device according to the first embodiment of the present invention;

Fig. 11 is a flowchart illustrating processing operation to be executed correspondingly until normalized interpersonal relationship evaluation values as to the respective partners are calculated based on the information of a target point in time which has been set, as processing operation to be executed at the server device according to the first embodiment of the present invention;

Fig. 12 is a flowchart illustrating processing operation to be executed at the time of performing operation for calculating and transmitting interpersonal relationship evaluation values at the server device side based on a request from the terminal device side, and performing operation for presenting interpersonal relationship at the terminal device side based on the transmitted interpersonal relationship evaluation values, as processing operation to be executed at the server device according to the first embodiment of the present invention;

Fig. 13 is a block diagram illustrating the internal configuration of a terminal device according to a second embodiment of the present invention; and

Fig. 14 is a flowchart illustrating processing operation to be executed at the time of setting a target point in time to be evaluated for calculating a mean value of distance, as processing operation to be executed at the server device according to the second embodiment of the present invention.

Preferred embodiments for carrying out the present invention will be described below.

First Embodiment

Overview of Interpersonal Relationship Evaluation System

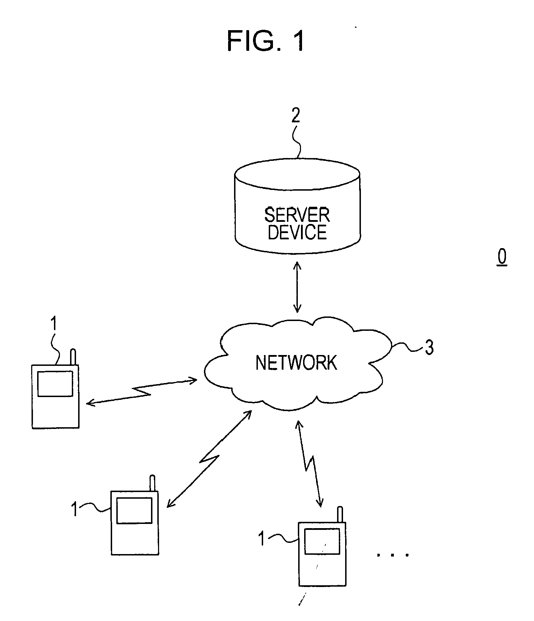

Fig. 1 is a diagram for describing overview of an interpersonal relationship evaluation system 0 serving as an interpersonal relationship evaluation system according to an embodiment of the present invention.

In Fig. 1, the interpersonal relationship evaluation system 0 according to the present embodiment is configured so as to include multiple terminal devices 1, a server device 2, and a network 3. The terminal devices 1, and server device 2 each have a network connection function, can perform data communication mutually through the network 3.

The terminal devices 1 have portability, and are assumed to be employed outdoors. In the case of the first embodiment, the terminal devices 1 include, for example, a GPS (Global Positioning System) reception unit, thereby enabling detection of a current position. Also, the terminal devices 1 include, for example, a direction sensor, thereby enabling detection of directions where the terminal devices 1 direct. For example, the terminal devices 1 include an acceleration sensor, thereby enabling detection of gravitational acceleration to be applied to the terminal devices 1. Such a terminal device 1 can be configured as, for example, a cellular phone, PDA (Personal Digital Assistant), personal computer (a type having portability such as a note type or the like), audio player, digital camera, or the like.

Various types of information such as position information, direction information (direction which a user turns to), and acceleration information (behavior of a user) detected at the respective terminal devices 1 are transmitted to the server device 2 sequentially, and information regarding the current position, direction, and behavior for each of the terminal devices 1 is managed for each point in time. As described later, with the interpersonal relationship evaluation system 0 of the present example, the interpersonal relationship between terminal device 1 users is evaluated based on the information thus managed by the server device 2.

Configuration Example of Terminal Devices

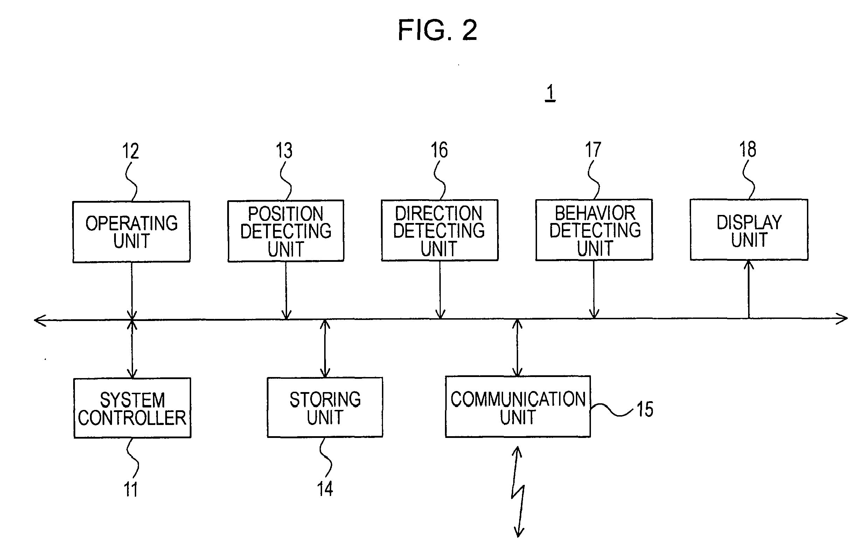

Fig. 2 is a block diagram illustrating the internal configuration of the terminal devices 1 shown in Fig. 2. As shown in the drawing, the terminal devices 1 include a system controller 11, operating unit 12, position detecting unit 13, storing unit 14, communication unit 15, direction detecting unit 16, behavior detecting unit 17, and display unit 18.

The system controller 11 is configured of a microcomputer including, for example, a CPU (Central Processing Unit), ROM (Read Only Memory), RAM (Random Access Memory), nonvolatile memory unit, and interface unit, which is a control unit for controlling the overall of the terminal device 1.

The system controller 11 controls each unit within the terminal device 1 to execute requested operation based on an operation program stored in a storing unit, for example, such as the above-mentioned ROM.

Also, in the case of the present example, ID information (UID: User ID) assigned so as to be unique for each of the terminal devices 1 is stored in the internal ROM or nonvolatile memory of the system controller 11. Thus, unique ID information is stored in each of the terminal devices 1, thereby enabling discrimination of each of the terminal devices 1 at the server device 2 side.

The operating unit 12 is provided as an operable member such as a key, dial, or the like for allowing a user who employs the terminal device 1 to perform various types of operations. For example, power on/off operations, operations for instructing start of various types of operation, operations for various types of settings, and so forth can be performed. The system controller 11 performs predetermined control processing based on the operation information from the operating unit 12.

The position detecting unit 13 is configured so as to include, for example, a GPG reception unit, and detects the current position of the terminal device 1. In this case, the GPS reception unit receives electric waves from an unshown GPS satellite, and detects information of latitude (x here), longitude (y here), and altitude (z here) as the above-mentioned current position to output these to the system controller 11.

The storing unit 14 performs recording (storing) of various types of data, or playback (reading) of recorded data based on the control of the system controller 11.

The storing unit 14 may configured of solid-state memory such as RAM or flash memory or the like, e.g., may be configured of an HDD (Hard Disk Drive).

Alternatively, the storing unit 14 may be configured of a recording/playback drive compatible with a recording medium such as a portable recording medium, e.g., a memory card having solid-state memory built-in, optical disc, magneto-optical disk, hologram memory, or the like instead of a built-in recording medium. It goes without saying that both of built-in type memory such as solid-state memory, HDD, or the like, and a recording/playback drive corresponding to a portable recording medium may be implemented.

The communication unit 15 performs transmission/reception of data with an external device. A unit may be employed as the communication unit 15 as long as the unit is subjected to network connection by wireless to perform communication.

The direction detecting unit 16 is configured of, for example, a direction sensor (magnetic field sensor), which detects a direction (orientation) which the terminal device 1 turns to. Note that if we make a comment for the sake of confirmation, the terminal device 1 is a device carried by a user, and accordingly, the direction information detected by the direction detecting unit 16 can be handled as the information of a direction which the user turns to. The direction information detected by the direction detecting unit 16 is supplied to the system controller 11.

The behavior detecting unit 17 is configured of, for example, an acceleration sensor, which detects gravitational acceleration applied to the terminal device 1. The acceleration information detected by the behavior detecting unit 17 can be handled as information representing the behavior of a user. The acceleration information detected by the behavior detecting unit 17 is also supplied to the system controller 11.

The display unit 18 is configured of, for example, a display device such as a liquid crystal display or the like, which performs display of various types of information based on the control of the system controller 11.

Configuration Example of Server Device

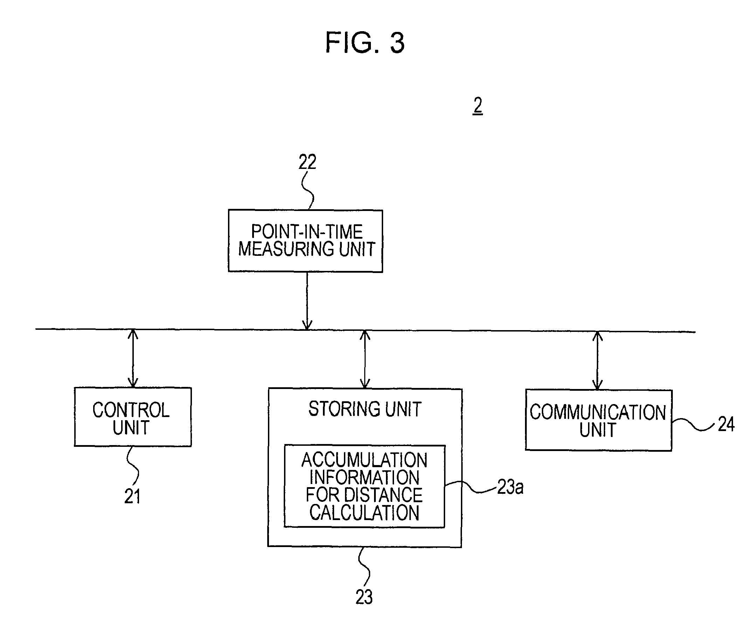

Fig. 3 is a block diagram illustrating the internal configuration of the server device 2 shown in Fig. 1. As shown in the drawing, the server device 2 includes a control unit 21, point-in-time measuring unit 22, storing unit 23, and communication unit 24. In Fig. 3, the control unit 21 performs the entire control of the server device 2. The control unit 21 is configured of CPU, ROM, RAM, nonvolatile memory unit, interface unit, and so forth, performs a requested calculation, and controls each unit within the server device 2 to execute requested operation based on an operation program stored in a storing unit, for example, such as the above-mentioned ROM.

The point-in-time measuring unit 22 measures the current point in time, for example, in a form of yyyy/mm/dd/hh/mm/ss, and outputs the obtained current point-in-time information to the control unit 21.

The storing unit 23 performs recording or playback of various types of data based on the control of the control unit 21.

Similarly, the storing unit 23 may be configured of solid-state memory such as RAM, flash memory, or the like, e.g., may be configured of an HDD (Hard Disk Drive). Alternatively, the storing unit 23 may be configured of a recording/playback drive compatible with a recording medium such as a portable recording medium, e.g., a memory card having solid-state memory built-in, optical disc, magneto-optical disk, hologram memory, or the like instead of a built-in recording medium. Alternatively, both of built-in type memory such as solid-state memory, HDD, or the like, and a recording/playback drive corresponding to a portable recording medium may be implemented.

With the present example, the storing unit 23 is employed as a storing unit of accumulation information for distance calculation 23a.

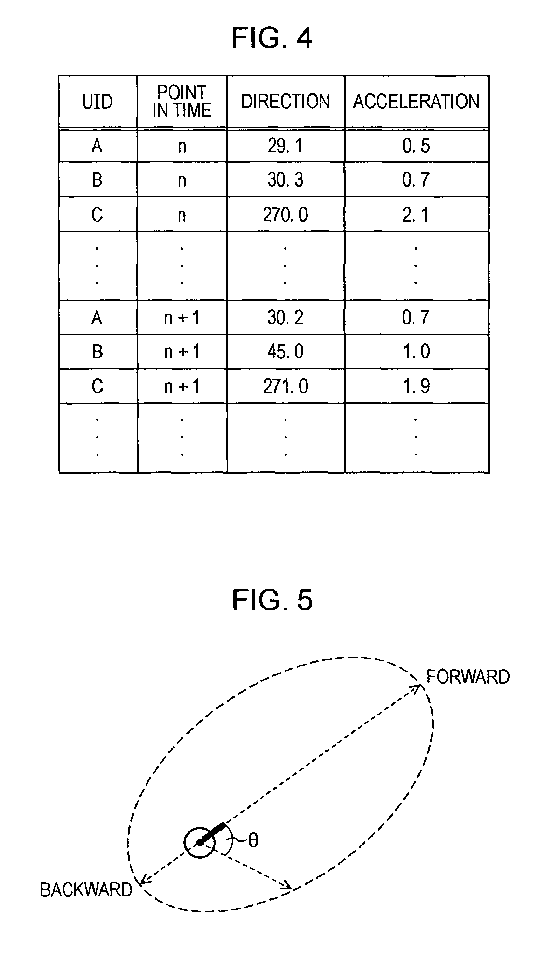

Now, description will be made regarding the accumulation information for distance calculation 23a with reference to the next Fig. 4.

As shown in Fig. 4, the accumulation information for distance calculation 23a is correlated with information of point-in-time, position, orientation, and acceleration for each UID.

The UID is unique ID information stored in each of the terminal devices 1 in the way described above. That is to say, with the server device 2 (control unit 21), according to the accumulation information for distance calculation 23a having a structure such as shown in this drawing, for each of the terminal devices 1, information of current position, direction, and acceleration (behavior) thereof can be managed for each point in time wherein such information was obtained.

Note that description will be made later regarding accumulation processing of such accumulation information for distance calculation 23a including obtaining of various types of information from each of the terminal devices 1.

Now, description will return to Fig. 3. The communication unit 24 performs transmission/reception of data with an external device. A unit can be employed as the communication unit 23 of the server device 2 as long as the unit is basically subjected to network connection by cable to perform communication, but may be configured so as to be subjected to network connection by wireless.

Evaluation Method of Interpersonal Relationship

With the interpersonal relationship evaluation system 0 according to the present embodiment, evaluation is performed regarding interpersonal relationship between the respective users of the terminal devices 1. In this case, interpersonal relationship evaluation values are obtained with a certain user X as reference regarding interpersonal relationship from the user X to each of other users. That is to say, with interpersonal relationship evaluation, it is fundamental to perform interpersonal relationship evaluation regarding interpersonal relationship from a target person to each of others.

Description will be made below regarding an interpersonal relationship evaluation method according to the first embodiment with reference to Figs. 5 through 9.

Note that description will be made below regarding a two person's relation between a user X (self, meaning the first person from the perspective from the primary user regarding which description is being made, referred to simply as "self" regardless of common English expressions for the sake of simplicity in the description) and a user A (partner) for convenience of explanation.

Condition setting according to target ranges

First, with the present embodiment, it is assumed that a condition setting will be performed for selecting a state to be evaluated for calculating an evaluation value. Specifically, with the first embodiment, it is assumed that conditions will be set regarding a range set with the user X self as reference, behavior (motion) of the users X and A, and a placement state of the users X and A.

First, description will be made regarding a target range setting method with reference to Fig. 5.

Such a target range setting is based on social distance concept of noncontact animals according to ethology. That is to say, even if interpersonal distance becomes an interpersonal relationship evaluation index, in a case wherein a partner is apart from the self by distance exceeding distance wherein the existence of the partner can be felt, interpersonal relationship does not depend on the distance of each person. Therefore, with the self as reference, a predetermined range is set wherein the existence of the partner can be felt such as described above, and a state wherein the partner exists within the range thereof is a target to be evaluated for calculating an evaluation value.

Now, such a target range can be set, for example, as a range within radius predetermined distance with the self's position as reference, but with the present embodiment, a target range is set so as to change distance depending on an angle θ with a direction which the self turns to as reference based on the distance concept wherein the partner can be felt has anisotropy similar to personal space of space behavior.

In Fig. 5, a circle mark within the drawing indicates the user X (self), and a solid line appended to the circle mark indicates a direction (front side) which the user X turns to, but as shown in this drawing, a target range in this case is set such that as the angle θ from the front side is shifted, distance gets shorter. Specifically, a target range is set such that as distance is gradually reduced from the θ = 0° to θ = 180°, distance at θ = 180° (direction right behind of the user X) becomes the shortest.

Such a target range can be set based on the position information and direction information obtained from the terminal device 1 carried by the user X. That is to say, if the position and direction are thus determined, determination is made whether to dispose the target range according to the angle θ, as described above, with the front direction which the user turns to as reference to which position in which shape.

As described above, a target to be evaluated for calculating an evaluation value is narrowed down to a state wherein the partner exists within the target range, whereby calculation of an evaluation value can be prevented from being performed with a state wherein existence of the partner is not felt, i.e., a state wherein no influence is applied to interpersonal relationship as a target, and accordingly, more accurate interpersonal relationship evaluation can be performed.

Condition setting according to behavior

Also, with the present embodiment, condition settings based on the behavior of each user are performed.

Specifically, "resting", "walking", and "running" are defined as the patterns of a user's behavior, and a state wherein both to be observed perform the same pattern of behavior is taken as a target to be evaluated for calculating an evaluation value.

For example, even if a person to be observed exists within the above-mentioned target range, in the case of passing through near a person who is simply resting, it can be estimated that the relationship of both is not so intimate. Accordingly, an evaluation value is calculated with a state wherein the behavior patterns of both are synchronized as a target.

Specifically, determination whether or not the patterns of behavior are synchronized can be performed based on acceleration information. In a case wherein a user act carrying the terminal device 1 for detecting acceleration such as the present example, acceleration waveform patterns upon which the respective states of "resting", "walking", and "running" are reflected are obtained, respectively. Accordingly, the acceleration waveform patterns obtained from the respective terminal devices 1 carried by the respective users to be observed are determined whether to match the above-mentioned classifications of "resting", "walking", and "running", whereby determination can be made whether or not the behavior patterns of the respective users to be observed are synchronized.

At this time, as described above, as a specific technique for determining whether or not the behavior patterns of both are matched with the classifications of "resting", "walking", and "running", a technique can be employed wherein determination is made whether or not the acceleration waveform pattern is matched with which waveform pattern defined as "resting", "walking", and "running" for each user, and determination is made whether or not mutually the same behavior pattern has been determined.

Condition setting according placement states

Also, with the present embodiment, further a condition regarding a placement state of the users to be observed is also set. Specifically, in this case, in order to obtain the interpersonal relationship evaluation value as to the user A (partner) from the user X (self), a case wherein the self's placement state as to the partner is a predetermined placement state is taken as a target to be evaluated for calculating an evaluation value.

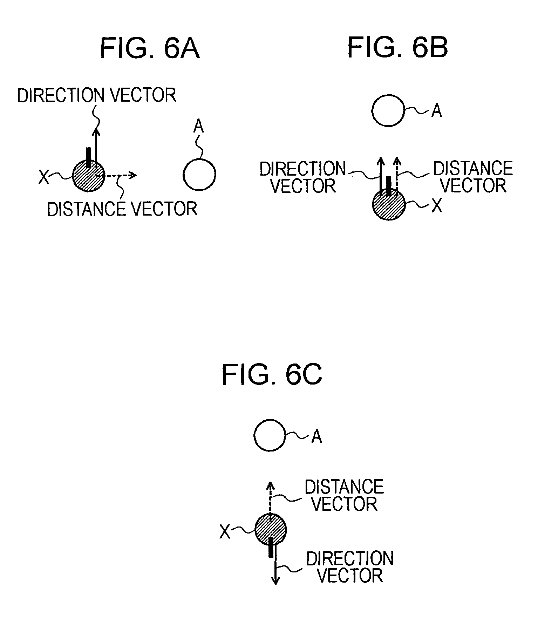

Specifically, three of "horizontal array" (Fig. 6A), "facing" (Fig. 6B), and "backside" (Fig. 6C) shown in the next Figs. 6A through 6C are defined as placement states. Note that in Figs. 6A through 6C as well, a circle mark within the drawing indicates the user X (self), and a solid line appended to the circle mark indicates a direction (front side) which the user X turns to. In these drawings, with regard to the user X (self) and user A (partner) to be observed, the user X

6

is indicated with a colored circle, and the user A is indicated with a white circle.

Now, in the case of the present example, the distance concept is also applied to any placement state shown in Figs. 6A through 6C. That is to say, even if only the array states shown in Figs. 6A through 6C hold, when the distance of both is apart, it is difficult to conclude that both have strong interpersonal relationship. Therefore, the distance between the self and partner is taken into consideration.

Specifically, when describing the condition of each placement state, first, with regard to "horizontal array" (Fig. 6A), a case is taken as a condition wherein the user X who is the self is arrayed horizontally as to the user A who is the partner, and mutual distance thereof is within predetermined distance.

Now, the placement state in this case is conceived with the user X who is the self as reference, so determination can be made based on the direction which the user X self turns to, and the position relationship between the users X and A whether or not the user X is arrayed horizontally as to the user A. Specifically, as shown in the drawing, if a relation holds wherein the direction which the user X self turns to is generally orthogonal to the direction where the user A exists as viewed from the user X, the user X is in a state arrayed horizontally as to the user A.

Similarly, with regard to "facing" (Fig. 6B), a case can be taken as a condition wherein the direction which the user X (self) turns to is generally identical to the direction where the user A exists as viewed from the user X, and mutual distance thereof is within predetermined distance. Determination is made regarding whether or not this condition holds, whereby determination can be made regarding whether or not the user X who is the self is in a "facing" state as to the user A who is the partner (strictly, whether or not the user X faces the user A, and mutual distance thereof is within predetermined distance).

Also, with regard to "backside" (Fig. 6C), in a direction opposite to "facing" (Fig. 6B), a case can be taken as a condition wherein the direction which the user X self turns to is generally the opposite direction as to the direction where the user A exists as viewed from the user X, and mutual distance thereof is within predetermined distance. Determination is made regarding whether or not this condition holds, whereby determination can be made regarding whether or not the user X who is the self is in a "backside" state as to the user A who is the partner (having the user X's back to the user A).

Now, with "horizontal array" shown in Fig. 6A, a situation is assumed wherein two having a friendly relationship are talking with adjacent seats, for example.

Also, with "facing" shown in Fig. 6B as well, a situation is assumed wherein two having a friendly relationship are talking, for example. Note that this "facing" is expected to include a state wherein the respective users have a conflict (e.g., verbal dispute or the like). However, even in such a conflict state, having strong interpersonal relationship remains unchanged, and the fact remains that appropriate interpersonal relationship evaluation can be performed.

Also, with "backside" shown in Fig. 6C, a situation is assumed wherein lovers are talking in a back-to-back manner, for example. In this case, if the distance between both is apart, in a case wherein this state is employed as a target to be evaluated for calculating an interpersonal relationship evaluation value, there is a possibility that it is difficult to perform appropriate evaluation. Here, as described above, an arrangement is made wherein the distance concept is also taken into consideration, whereby in a state wherein both are approaching with a certain level or more in such a back-to-back state can be taken as a target, thereby obtaining an appropriate evaluation value.

Description will be made specifically regarding the conditions of these placement states, and a method for determining whether or not the conditions hold.

First, let us say that the distance between both to be observed is obtained with the following Expression 1. In the following expression, let us say that both to be observed are the users X and A, $(x_X, y_X, z_X)$ represents the position of the user X, and $(x_A, y_A, z_A)$ represents the position of the user A, respectively.

[Expression 1]

$$d_{XA} = \sqrt{(x_X - x_A)^2 + (y_X - y_A)^2 + (z_X - z_A)^2}$$

With regard to "horizontal array",
$\alpha$ is a threshold for determining "horizontal array", and
$\beta$ is the limited distance of "horizontal array",
and the distance $d_{XA}$ obtained from the above-mentioned Expression 1, these $\alpha$ and $\beta$, and further a direction vector (solid line arrow in the drawing) and distance vector (dashed line arrow in the drawing) shown in Fig. 6, are employed, thereby determining whether or not a condition holds.

Now, the above-mentioned direction vector is equivalent to the direction which the user X who is the self turns to, and is obtained by normalizing the direction information obtained from the terminal device 1 carried by the user X.

Also, the above-mentioned distance vector is equivalent to the direction from the position of the user X to the position of the user A, and is obtained based on the position information obtained from the terminal device 1 of the user A.

In this case, the direction vector and distance vector employ different information as a source, so values obtained by normalizing both are employed.

An inner product dp between the direction vector and distance vector is employed, whereby determination can be made whether or not the user X is in a state arrayed horizontally as to the user A. That is to say, if

$$dp < \alpha,$$

determination can be made whether or not the user X is in a state horizontally arrayed as to the user A. Also, in this case, distance is also taken into consideration, so determination is made regarding whether or not the present state is a "horizontal array" state by determining regarding whether or not the following conditions hold.

$$dp < \alpha \ \text{and} \ d_{XA} < \beta$$

Similarly, determination is made whether or not conditions regarding "facing" and "backside" hold.

With regard to "facing", a state wherein the self turns to the direction where the partner exists can be included. That is to say, a value including such a state can be set as a threshold for determining "facing". Specifically, in this case, let us say that
0.5 is a threshold for determining "facing", and
$\gamma$ is the limited distance of "facing",
determination is made regarding whether or not the following conditions hold.

$$dp < 0.5 \ \text{and} \ d_{XA} < \gamma$$

Also, with regard to "backside", a state wherein the self turns to the opposite direction of the direction where the partner exists can be included, and in this case, let us say that
-0.5 is a threshold for determining "backside", and
$\delta$ is the limited distance of "backside",
determination is made regarding whether or not the following conditions hold.

$$dp < -0.5 \ \text{and} \ d_{XA} < \delta$$

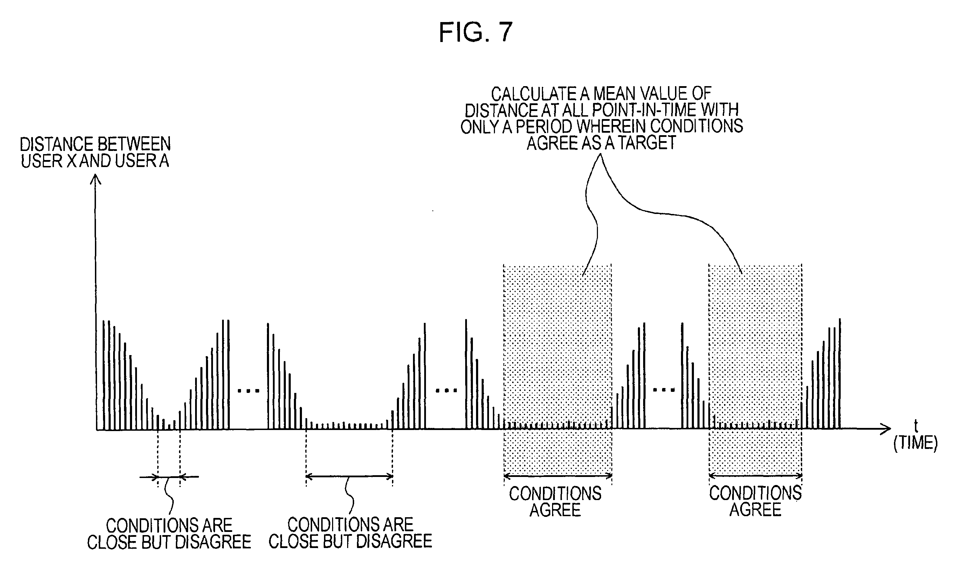

Fig. 7 is a diagram for describing an entire flow at the time of calculating an interpersonal relationship evaluation value.

In Fig. 7, the horizontal axis is taken as time (t), the vertical axis is taken as distance $d_{XA}$ between the users X and A, and exemplifies a situation wherein the distance $d_{XA}$ is changed with time.

As shown in Fig. 7, with the present embodiment, as a result of the above-mentioned determination regarding whether or not each condition holds, only a period wherein each condition has been determined to be matched is taken as a target, and a mean value of distance at all points in time within the period thereof is calculated. That is to say, thus, an interpersonal relationship evaluation value for evaluating interpersonal relationship is calculated.

Specifically, determination regarding whether or not each condition is matched is performed for each point in time based on the position information, direction information, and acceleration information obtained for each point in time from each of the terminal devices 1. Subsequently, as a result of such determination for each point in time, i.e., a point in time wherein each condition has been determined to be matched is set as a point in time to be evaluated for calculation, and a mean value of the distance $d_{XA}$ between the users X and A at each point in time to be evaluated for calculation is calculated, thereby obtaining a mean value of the $d_{XA}$ as an interpersonal relationship evaluation value of the user X as to the user A. System Operation Explanation

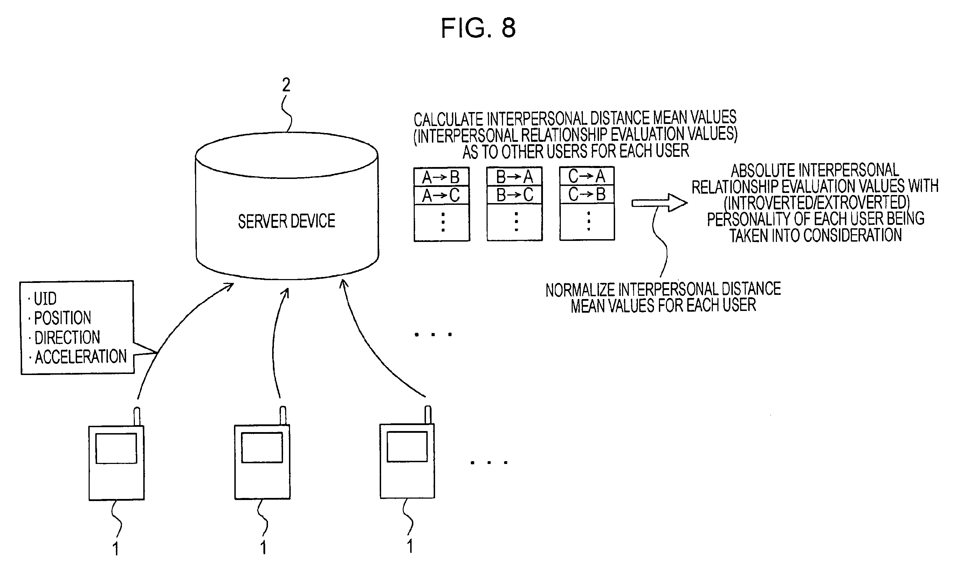

Fig. 8 is a diagram schematically illustrating operation overview of the interpersonal relationship evaluation system 0 according to the first embodiment performed base on the interpersonal relationship evaluation value calculation method according to the first embodiment described above. Note that in this drawing, the network 3 shown in Fig. 1 is omitted. In Fig. 8, each of the terminal devices 1 transmits the position information, direction information, and acceleration information obtained at each point in time to the server device 2 successively. Also, in addition to such information, each of the terminal devices 1 transmits UID information to the server device 2.

The server device 2 receives the UID, position information, direction information, and acceleration information transmitted from each of the terminal devices 1, and records these in the storing unit 23 so as to accumulate these as accumulation information for distance calculation 23a shown in Fig. 4.

Now, description will be made regarding transmission of such various types of information to the sever device 2 side from the terminal device 1 side, and control processing of the server device 2 at the time of accumulating various types of information.

With the terminal device 1 side, the system controller 11 obtains the position information, direction information, and acceleration information detected at each point in time at the position detecting unit 13, direction determining unit 16, and behavior detecting unit 17, respectively. Subsequently, the system controller 11 controls the communication unit 15 to transmit the position information, direction information, and acceleration information thus obtained, and the UID information stored in memory such as internal ROM or the like to the server device 2 successively.

With the server device 2 side, upon such various types of information being transmitted from each of the terminal devices 1 by the communication unit 24, the control unit 21 performs control such that such various types of information is correlated with the current point-in-time information measured by the point-in-time measuring unit 22, and recorded in the storing unit 23. Thus, the accumulation information for distance calculation 23a is formed such as shown in Fig. 4.

Returning to the previous topic, with the server device 2, the control unit 21 performs calculation of an interpersonal relationship evaluation value based on the above-mentioned accumulation information for distance calculation 23a. As described above, with regard to interpersonal relationship evaluation values, it is fundamental to obtain an interpersonal relationship evaluation value with a two person's relation between the self and the partner. Such a calculation between two persons is also performed regarding a different partner in the same way, whereby an interpersonal relationship evaluation value of a single user to be observed as to each of the others can be obtained. Subsequently, such a calculation of an interpersonal relationship evaluation value regarding a single user as to each of the other users is performed regarding each of the other users in the same way, whereby an interpersonal relationship evaluation value of each of the users as to each of the users can be obtained.

Description will be made regarding calculation operation of an interpersonal relationship evaluation value between two persons, which is the most fundamental level for performing interpersonal relationship evaluation according to the present example.

Such as described previously, in order to obtain a mean value of distance serving as an interpersonal relationship evaluation value, determination can be performed regarding whether or not the above-mentioned various types of condition hold for each point in time.

First of all, determination regarding the above-mentioned target range is performed. That is to say, with regard to a point in time n to be determined, the position information of the user X (UID-X) serving as the self, the position information of the user A (UID-A) serving as the partner, and the direction information of the user X are obtained from the accumulation information for distance calculation 23a. Subsequently, a target range is set such that distance is shortened depending on the angle θ from the direction indicated with the direction information in the way described above based on the position information and direction information of the user X, and determination is made based on the information of the target range thereof, and the position information of the user A serving as the partner whether or not the relevant user A exists within the target range thereof.

In a case wherein determination has been made that the condition regarding the above-mentioned target range holds, determination is made regarding whether or not the condition according to behavior holds. That is to say, the acceleration information (i.e., acceleration waveforms) of the users X and A during a predetermined period is obtained with the point in time n as reference, and determination is made whether or not these waveform patterns are matched with the above-mentioned classifications of "resting", "walking", and "running".

In a case wherein determination has been made that the above-mentioned condition according to behavior holds, further determination is made regarding whether or not the condition according to a placement state holds. That is to say, distance $D_{XA}$ is obtained by the calculation shown in the previous Expression 1 from the position information of the users X and A at the point in time n obtained as described above, and the direction vector and distance vector described with reference to Fig. 6 are obtained by calculation. The direction vector can be calculated based on the direction information of the user X obtained as described above, and the distance vector can be calculated from the position information of the users X and A. Moreover, the inner product dp between the direction vector and distance vector is calculated.

Subsequently, the values of the distance $d_{XA}$ and inner product dp, and the values of the $\alpha$ (threshold for determining horizontal array), $\beta$ (limited distance of horizontal array), $\gamma$ (limited distance of facing), and $\delta$ (limited distance

of backside) which have been set beforehand are employed to perform the above-mentioned determinations are made regarding whether or not the respective conditions hold, thereby determining regarding whether or not the placement state of the user X as to the user A is matched with any of the "horizontal array", "facing", and "backside".

As a result of the determinations regarding these placement states, in a case wherein determination has been made that the placement state of the user X as to the user A is matched with any of the "horizontal array", "facing", and "backside", determination is made that all of the conditions with the present example are satisfied, the point in time n serving as a determination target is set as a point in time to be evaluated for calculating an evaluation value.

Operation regarding one point-in-time worth described above is performed regarding all points in time during the period set as an evaluation target, thereby obtaining all points in time to be evaluated for calculating a mean value of distance serving as an evaluation value. A mean value of distance between the users X and A is calculated with a point in time to be evaluated for calculating a mean value thus obtained as a target. Specifically, the value of the distance $d_{XA}$ between the users X and A at each point in time to be evaluated for calculating a mean value is all added, the value thereof is divided by the number of points in time to be evaluated for obtaining a mean value, thereby obtaining a mean value of interpersonal distance representing interpersonal relationship of the user X as to the user A.

Thus, an interpersonal relationship evaluation value from one to the other between certain two persons within a certain target period can be obtained. In the way described above, such a calculation of an interpersonal relationship evaluation value between two persons is performed by changing the partner and the self, whereby an interpersonal relationship evaluation value of each person as to each person can be calculated.

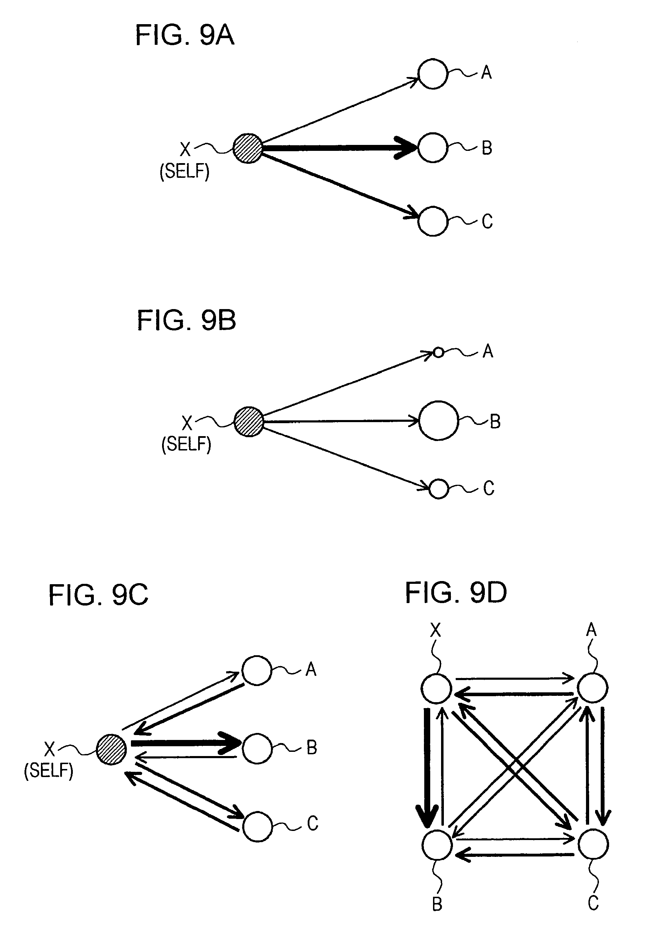

Now, Figs. 9A through 9D illustrate a display (presentation) example of an evaluation result of interpersonal relationship.

First, Figs. 9A and 9B exemplify a presentation technique of an evaluation result of interpersonal relationship of the certain user X (self) as to other respective users (three persons of A, B, and C, here). In order to realize such a presentation technique, only interpersonal relationship evaluation values from the user X serving as the self to the other respective users A, B, and C have to be calculated. At this time, the interpersonal relationship expression technique can be represented with the thickness of an arrow as to the respective partner side users as shown in Fig. 9A.

Alternatively, as shown in Fig. 9B, interpersonal relationship can be represented with the size of a mark representing the respective users on the partner side.

Alternatively, as shown in Fig. 9C, an interpersonal relationship evaluation result as to a user serving as the self from each user on the partner side may be presented together. In this case, evaluation value calculation of the user serving as the self as to each user of the partner side, and evaluation value calculation as to the user serving as the self from each user on the partner side have to be performed. Fig. 9C illustrates an example wherein interpersonal relationship is represented with the thickness of an arrow in the same way as in Fig. 9A.

Alternatively, as shown in Fig. 9D, an interpersonal relationship evaluation result of each user to be evaluated as to each user may be presented, which is a so-called person correlation diagram. In this case, an interpersonal relationship evaluation value can be calculated regarding the users to be evaluated (X, A, B, and C in the drawing) in a round-robin manner. In this case as well, a case wherein each interpersonal relationship evaluation result is represented with the thickness of an arrow is exemplified.

Now, as can be understood from the above description, an interpersonal relationship evaluation value according to the present embodiment is equivalent to a mean value of interpersonal distance as to the partner. That is to say, the final evaluation index is "interpersonal distance".

At this time, it is projected that a scale regarding near interpersonal distance/far interpersonal distance differs depending on the personality of each user, e.g., an introvert person or extravert person. Specifically, it is projected that while an extravert person can perform near communication as to others, an introvert person is not good at near communication as to others.

Accordingly, with regard to unilateral interpersonal relationship as to each of the partners from a certain user serving as the self, an evaluation value of the present example with distance as an evaluation index can be regarded as an absolute evaluation index, but for example, when presenting evaluation results from others together such as shown in Figs. 9C and 9D, there is a possibility that scales between the respective users differ, and it is difficult to perform absolute evaluation.

Therefore, as shown in Fig. 8 as well, a technique is employed here wherein interpersonal relationship evaluation values (interpersonal distance mean values) calculated regarding a certain user serving as the self are normalized.

Specifically, the interpersonal distance mean values as to other respective users obtained regarding a certain user are all added, and the interpersonal mean value as to each user is divided by the obtained value, thereby performing normalization. That is to say, for example, if we say that the interpersonal distance mean values of the user X as to the respective users are $D_{XA}$, $D_{XB}$, and $D_{XC}$ when assuming that the self is the user X, the partners are the users A, B, and C, a total interpersonal distance DX which is a total value thereof is calculated as follows.

$$DX = D_{XA} + D_{XB} + D_{XC}$$

Moreover, absolute interpersonal relationship evaluation values $D^*_{XA}$, $D^*_{XB}$, and $D^*_{XC}$ after normalization are calculated as follows.

$$D^*_{XA} = D_{XA} / D_X$$

$$D^*_{XB} = D_{XB} / D_X$$

$$D^*_{XC} = D_{XC} / D_X$$

Normalization such as described above is performed, whereby the evaluation values can be converted into values equivalent to the percentages occupied by the respective partners with the interpersonal relationship of the self. That is to say, thus, difference of interpersonal distance scales of the respective users can be eliminated, the interpersonal relationship of each user can be evaluated in an absolute manner.

Processing Operation

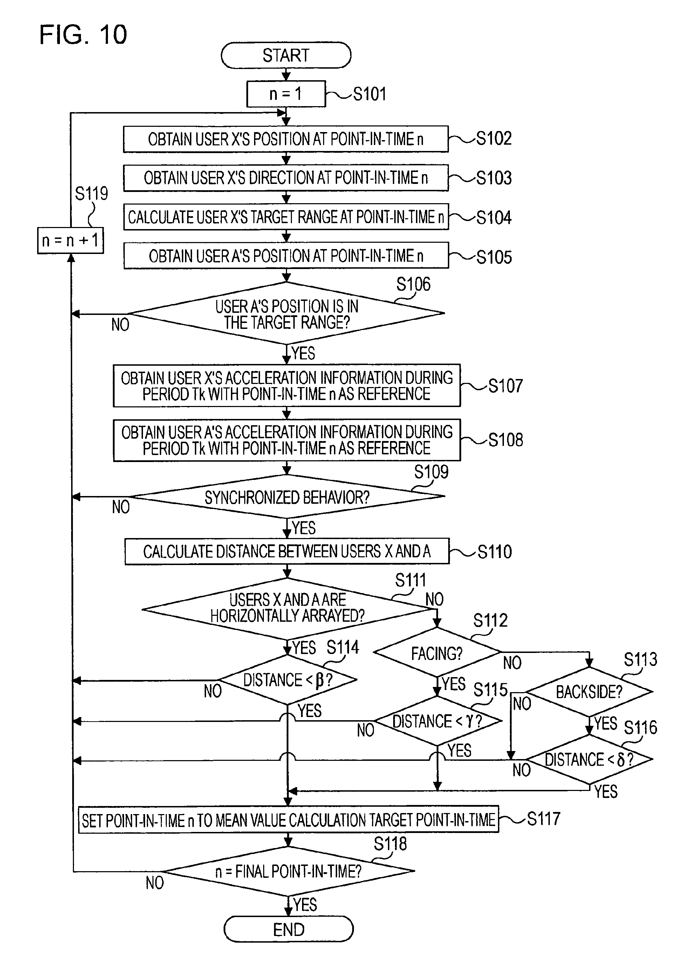

Subsequently, description will be made regarding processing operation to be executed to realize the operation of the interpersonal relationship evaluation system 0 according to the first embodiment described above with reference to the flowcharts shown in Figs. 10 through 12.

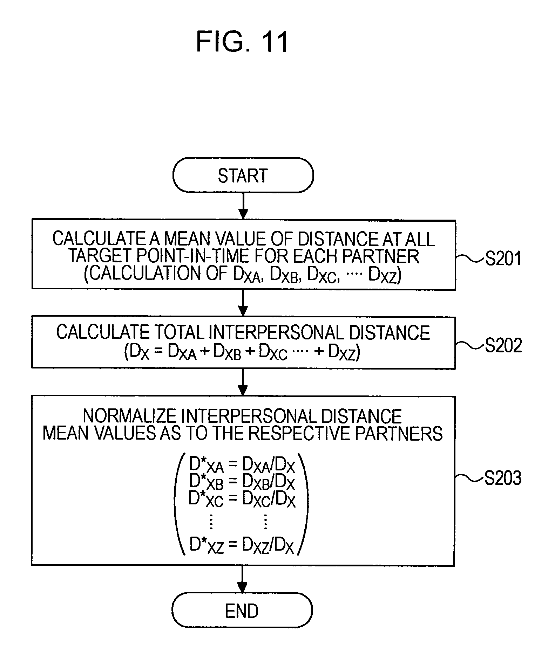

First, Figs. 10 and 11 illustrate processing operation to be executed correspondingly until the server device 2 calculates normalized interpersonal relationship evaluation values. The processing operation shown in Figs. 10 and 11 is executed by the control unit 21 shown in Fig. 3 based on a program stored in internal ROM or the like.

Fig. 10 illustrates processing operation for setting a point in time to be evaluated for calculating an evaluation value (point in time to be evaluated for calculating an interpersonal distance mean value) based on the determination results regarding whether or not the above-mentioned various types of conditions hold.

Note here that a user serving as the self is represented with "X", and a user serving as the partner is represented with "A". This drawing illustrates processing operation for setting a point in time to be evaluated for calculating a distance mean value regarding a two person's relation between the self and a single partner, but such processing operation can be executed regarding each user to be evaluated as appropriate.

First, in step S101, processing for setting a point-in-time identification value to n = 1 is executed. This point-in-time identification value n is a value for identifying a point in time to be evaluated for various types of determination processing shown in this drawing, of the respective points in time with the accumulation information for distance calculation 23a shown in Fig. 4. For example, the oldest point in time with a period to be evaluated is set to n = 1 or the like, and the value of the n is incremented sequentially, thereby referencing each point in time within the target period sequentially.

In subsequent step S102, the position of the user X at the point in time n is obtained. That is to say, position information wherein the UID representing the user X, and point-in-time information serving as the point-in-time n are correlated is obtained within the accumulation information for distance calculation 23a accumulated in the storing unit 23.

In the next step S103, the direction of the user X at the point in time n is obtained. That is to say, direction information wherein the UID representing the user X, and point-in-time information serving as the point-in-time n are correlated is obtained from the accumulation information for distance calculation 23a.

In the next step S104, the target range of the user X at the point in time n is calculated. That is to say, a target range such as shown in Fig. 5 at the point in time n of the user X is obtained by calculation based on the position information and direction information obtained in the above-mentioned steps S102 and S103, respectively.

In subsequent step S105, the position of the user A at the point in time n is obtained. That is to say, position information wherein the UID representing the user A, and point-in-time information serving as the point-in-time n are correlated is obtained from the accumulation information for distance calculation 23a.

Moreover, in the next step S106, determination is made regarding whether or not the position of the user A is in the target range. In a case wherein a negative result has been obtained in step S106 wherein the position of the user A at the point in time n is not in the target range obtained in the above-mentioned step S104, the processing proceeds to step S119 as shown in the drawing, where the identification value n is incremented (n = n + 1), and then the processing returns to the previous step S102. That is to say, thus, the point in time n is excluded from the points in time to be evaluated for calculating a distance mean value.

On the other hand, in a case wherein a positive result has been obtained in the above-mentioned step S106 wherein the position of the user A at the point in time n is in the target range, the processing proceeds to step S107, where the acceleration information of the user X during a period Tk with the point in time n as reference is obtained. That is to say, of the acceleration information correlated with the user X of the accumulation information 23a for distance calculation, with a predetermined period before and after the point in time n serving as reference as a period Tk, acceleration information is obtained during the period Tk. Thus, information representing change in acceleration (acceleration waveform) during the period Tk is obtained.

In subsequent step S108, the acceleration information of the user A during the period Tk with the point in time n as reference is obtained. That is to say, of the acceleration information correlated with the user A in the accumulation information for distance calculation 23a, the acceleration information (acceleration waveform) during the period Tk with the point in time n as reference is obtained.

In the next step S109, determination is made regarding whether or not behavior is synchronized. That is to say, determination is made whether or not the acceleration waveform pattern regarding the user X obtained in the above-mentioned step S107, and the acceleration waveform pattern of the user A obtained in the above-mentioned step S108 are matched with any of the above-mentioned classifications of "resting", "walking", and "running".

In a case wherein a negative result has been obtained in step S109 wherein both waveform patterns are not matched, and behavior is not synchronized, the processing proceeds to step S119, where the identification value n is incremented, and then the processing returns to the previous step S102.

On the other hand, in a case wherein a positive result has been obtained in the above-mentioned step S109 wherein both waveform patterns are matched, and behavior is synchronized, the processing proceeds to step S110, where distance between the users X and A is calculated. That is to say, when assuming that the position of the user X at the point in time n (obtained in the previous step S102) is $(x_X, y_X, z_X)$, and the position of the user A at the point in time n (obtained in step S105) is $(x_A, y_A, z_A)$, the distance $d_{XA}$ from the user X to user A (interpersonal distance between the users X and A) is obtained by performing calculation by the previous Expression 1.

Note that the reason why interpersonal distance is obtained in the above-mentioned step S110 is because interpersonal distance can be employed for determination regarding whether or not the condition regarding the placement state holds with the processing in steps S111 and thereafter. Here, thus, the reason why the value of interpersonal distance is obtained immediately before determination regarding whether or not the condition regarding the placement state holds is because interpersonal distance is calculated in response to obtaining all positive results from determinations regarding whether or not the conditions regarding other target range and behavior state hold. Thus, in a case wherein wasteful calculation processing in a case wherein interpersonal distance has not to be obtained can be omitted, and consequently, reduction in processing load requested for setting of a point in time to be evaluated for calculation is realized.

In step S111 subsequent to the above-mentioned step S110, determination is made regarding whether or not the users X and A are in a horizontal array state. That is to say, after the inner product dp between the direction vector and distance vector described in the previous Fig. 6 is calculated, determination is made regarding whether or not the following condition holds based on the value of the inner product dp, and further α (threshold for determining horizontal array) which has been set beforehand.

$$dp < \alpha$$

In a case wherein a positive result has been obtained in step S111 wherein the above-mentioned condition holds, and the user X is in a horizontal array state as to the user A, the processing proceeds to step S114, where determination is further made regarding whether or not the distance < β. That is to say, determination is made regarding whether or not the following condition holds based on the value of the distance $d_{XA}$ calculated in step S110, and the value of the β (limited distance of horizontal array).

$$d_{XA} < \beta$$

In a case wherein a negative result has been obtained in step S114 wherein the above-mentioned condition does not hold, and the distance < β is not satisfied, the processing proceeds to step S119, where the identification value n is incremented, and then the processing returns to step S102.

On the other hand, in a case wherein a positive result has been obtained wherein the above-mentioned condition holds, and the distance < β is satisfied, the processing proceeds to later-described step S117.

Also, in a case wherein a negative result has been obtained in step S111 wherein the user X is not in a horizontal array state as to the user A, in step S112 determination is made regarding whether or not the user X in a facing state as to the user A. Specifically, a threshold for determining facing is set to 0.5, and determination is made regarding whether or not the following condition holds.

$$dp > 0.5$$

In a case wherein a positive result has been obtained in step S112 wherein the above-mentioned condition holds, and the user X is in a facing sate as to the user A, the processing proceeds to step S115, where determination is made regarding whether or not the distance < γ. Specifically, determination is made regarding whether or not the following condition holds based on the value of the distance $d_{XA}$ and the value of the γ (limited distance of facing).

$$d_{XA} < \gamma$$

In a case wherein a negative result has been obtained in step S115 wherein the above-mentioned condition does not hold, and the distance < γ is not satisfied, the processing proceeds to step S119, where the identification value n is incremented, and then the processing returns to step S102.

On the other hand, in a case wherein the above-mentioned condition holds, and the distance < γ is satisfied, the processing proceeds to step S117.

Also, in a case wherein a negative result has been obtained in step S114 wherein the user X is not in a facing state as to the user A, in step S113 determination is made regarding whether or not the user X in a backside state as to the user A. Specifically, a threshold for determining backside is set to -0.5, and determination is made regarding whether or not the following condition holds.

$$dp > -0.5$$

In a case wherein a positive result has been obtained in step S113 wherein the above-mentioned condition holds, and the user X is in a backside sate as to the user A, the processing proceeds to step S116, where determination is made regarding whether or not the distance < δ. Specifically, determination is made regarding whether or not the following condition holds based on the value of the distance $d_{XA}$ and the value of the δ (limited distance of backside).

$$d_{XA} < \delta$$

In a case wherein a negative result has been obtained in step S116 wherein the above-mentioned condition does not hold, and the distance < δ is not satisfied, the processing proceeds to step S119, where the identification value n is incremented, and then the processing returns to step S102.

On the other hand, in a case wherein the above-mentioned condition holds, and the distance < δ is satisfied, the processing proceeds to step S117.

In step S117, processing for setting the point in time n to a point in time to be evaluated for calculating a mean value is performed.

In subsequent step S118, determination is made regarding whether or not the n is a final point in time. Specifically, determination is made regarding whether or not the value of the point-in-time identification value n is a value representing the final point in time within a predetermined period as an evaluation target period.

In a case wherein a negative result has been obtained in step S118 wherein the n has not reached the final point in time, the processing proceeds to step S119, where the identification value n is incremented, and then the processing returns to step S102. On the other hand, in a case wherein a positive result has been obtained wherein the n has reached the final point in time, the processing operation shown in this drawing is ended.

Subsequently, Fig. 11 illustrates processing operation to be executed correspondingly until a normalized interpersonal evaluation value as to each partner is calculated based on the information of a point in time to be evaluated for calculating a mean value set by the processing operation shown in Fig. 10.

Fig. 11 illustrates processing for calculating an interpersonal relationship evaluation value as to each partner from a certain single user serving as the self, but can be understood from the previous description, such processing can be executed for each user serving as the self.

In Fig. 11, first, in step S201, a mean value of distance of all points in time to be referenced is calculated for each partner. Specifically, in this case as well, when assuming that the self is a user X, and the respective partners are users A, B, C, and so on through Z, a mean value of distance at all points in time to be referenced ($D_{XA}$, $D_{XB}$, $D_{XC}$, and so on through $D_{XZ}$) is calculated for each partner based on information of a point in time to be evaluated for calculating a mean value which has been set in the processing operation shown in Fig. 10 regarding the respective partners A, B, C, and so on through Z, and information of interpersonal distance ($d_{XA}$, $d_{XB}$, $d_{XC}$, and so on through $d_{XZ}$) at each point in time to be evaluated for calculating a mean value.

The subsequent steps S202 and S203 are processing for normalizing an interpersonal distance mean value as to each partner obtained in the processing in the above-mentioned step S201.

Specifically, first, in step S202, calculation of total interpersonal distance is performed. Specifically, total interpersonal distance $D_X$ is obtained with the following expression.

$$D_X = D_{XA} + D_{XB} + D_{XC} +, . . . + D_{XZ}$$

Moreover, in step S203, processing for normalizing a interpersonal distance mean value as to each partner is performed. Specifically, interpersonal relationship evaluation values $D^*_{XA}$, $D^*_{XB}$, $D^*_{XC}$, and so on through $D^*_{XZ}$ a as to each partner after normalization are obtained as follows. $D^*_{XA}$, $D^*_{XB}$, and $D^*_{XC}$ after normalization are calculated as follows.

$$D^*_{XA} = D_{XA} / D_X$$

$$D^*_{XB} = D_{XB} / D_X$$

$$D^*_{XC} = D_{XC} / D_X$$

and so on through

$$D^*_{XZ} = D_{XZ} / D_X$$

Upon the normalizing processing in step S203 being executed, the processing operation shown in this drawing is ended.

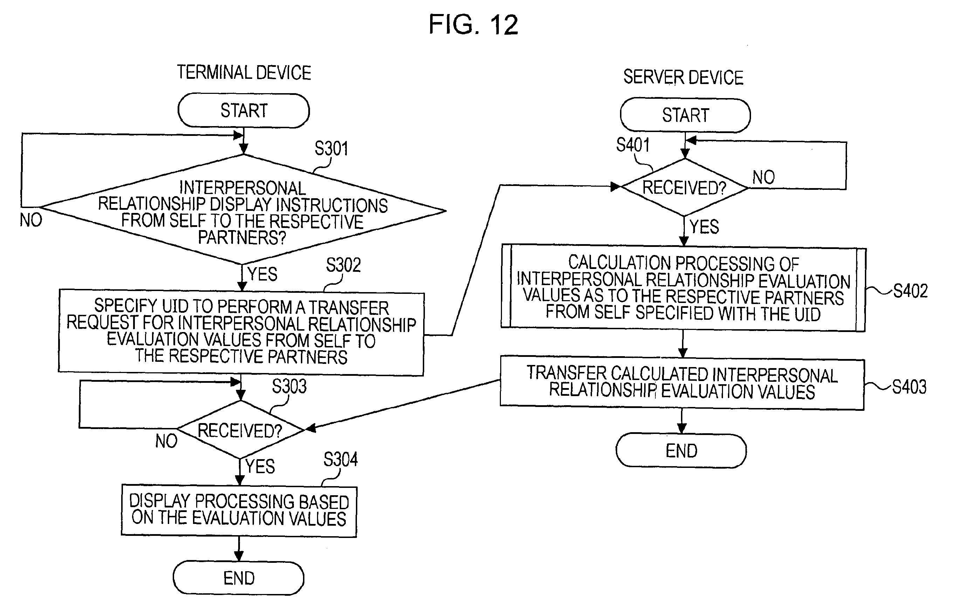

Fig. 12 illustrates operation wherein the server device 2 transmits an interpersonal relationship evaluation value to be calculated with the processing operation in Figs. 10 and 11 described above based on a request from the terminal device 1, and processing operation to be executed at the time of displaying interpersonal relationship at the terminal device 1 side based on the transmitted interpersonal relationship evaluation value.

Note that in Fig. 12, the processing operation shown as "terminal device" is processing operation which the system controller 11 shown in the previous Fig. 2 executes based on the program stored in, for example, internal ROM. Also, the processing operation shown as "server device" is processing operation which the control unit 21 shown in Fig. 3 executes based on the program stored in, for example, the above-mentioned ROM.

In Fig. 12, processing operation to be executed correspondingly in a case wherein display of interpersonal relationship as to each partner is instructed from the user X serving as the self at the terminal device 1 side, as an example.

First, in step S301, interpersonal relationship display instructions from the self to each partner are awaited at the terminal device 1 side. That is to say, the terminal device 1 is awaited until display instructions of interpersonal relationship as to each partner which has been set beforehand are performed from the user self of the relevant terminal device 1. Note that such display instructions are performed, for example, through the operating unit 12 shown in Fig. 2.

In a case wherein the above-mentioned display instructions are received, in step S302, the terminal device 1 side specifies the UID to perform a transfer request for interpersonal relationship evaluation values as to the respective partners from the self. Specifically, the terminal device 1 side controls the communication unit 15 to transmit information of the UID stored in the internal ROM or the like, and information for requesting for transfer of interpersonal relationship evaluation values as to the respective partners to the server device 2.

Upon executing the transfer processing in step S302, the processing proceeds to later-described step S303.

In step S401 in the drawing, the server device 2 side performs processing for awaiting that the information transferred in the above-mentioned step S302 is received at the communication unit 24. Subsequently, in a case wherein the above-mentioned transfer information from the terminal device 1 is received, in step S402 the server device 2 side executes calculation processing of interpersonal relationship evaluation values as to the respective partners from the self specified by the UID. Specifically, when assuming that the user specified with the received UID is the self X, and predetermined respective partners A, B, C, and so on through Z, the server device 2 side executes the processing operation described with reference to Figs. 10 and 11, thereby calculating normalized interpersonal relationship evaluation values $D^*_{XA}$, $D^*_{XB}$, $D^*_{XC}$, and so on through $D^*_{XZ}$.

In subsequent step S403, the server device 2 side executes transfer processing of the calculated interpersonal relationship evaluation values. Specifically, the server device 2 side controls the communication unit 24 to transmit the interpersonal relationship evaluation values $D^*_{XA}$, $D^*_{XB}$, $D^*_{XC}$, and so on through $D^*_{XZ}$ calculated in the processing in the above-mentioned step S402 to the terminal device 1.

Upon executing the processing in step S403, the processing operation on the server device 2 side shown in this drawing is ended.

In step S303, the terminal device 1 side awaits that the above-mentioned interpersonal relationship evaluation values calculated by the server device 2 are received by the communication unit 15. Subsequently, in a case wherein the above-mentioned interpersonal relationship evaluation values from the server device 2 are received, in step S304 the terminal device 1 side executes display processing based on the evaluation values. Specifically, the terminal device 1 side performs display control such that display representing interpersonal relationship as to the respective partners A, B, C, and so on through Z from the user X serving as the self is performed on the display unit 18, for example, according to a display mode such as shown in the previous Figs. 9A or 9B.

Upon executing the processing in step S304, the processing operation on the terminal device 1 side shown in this drawing is ended.

Note that Fig. 12 exemplifies a case wherein display instructions regarding unidirectional interpersonal relationship from a single user serving as the self to the respective partners are performed as an example, but in a case wherein display instructions of bidirectional interpersonal relationship from the self to the respective partners, and from the respective partners to the self are performed, for example, as shown in Fig. 9C, or even in a case wherein display instructions of bidirectional interpersonal relationship from each person to each person are performed, such as shown in Fig. 9D, the same processing as the processing shown in Fig. 12 can be executed basically.

For example, in a case wherein display instructions of bidirectional interpersonal relationship from the self to the respective partners, and the respective partners to the self are performed, such as shown in Fig. 9C, in step S303 the terminal device 1 side has to perform a transfer request for interpersonal relationship evaluation values from the self to the respective partners, and from the respective partners to the self, and in response to this, in step S402 the server device 2 side has to perform calculation of the interpersonal relationship evaluation values from the self to the respective partners, and from the respective partners to the self.

Similarly, in a case wherein display instructions of bidirectional interpersonal relationship from each person to each person are performed, such as shown in Fig. 9D, in step S302 the terminal device 1 side has to perform a transfer request of interpersonal relationship evaluation values from each person to each person, and in step S402 the server device 2 side has to perform calculation of interpersonal relationship evaluation values from each person to each person.

Here, with the above description, in response to a request from the terminal device 1 side, the server device 2 calculates requested interpersonal relationship evaluation values, but instead of this, an arrangement may be made wherein the server device 2 side calculates interpersonal relationship evaluation values of each person as to each person in a certain cycle such as each point in time or the like, and when receiving a request from the terminal device 1 side, make preparations so as to immediately transfer the interpersonal relationship evaluation values corresponding to the request.

However, if calculation of interpersonal relationship evaluation values is performed according to a request, calculation processing at the server device 2 can be suppressed to the requested relevant processing, and accordingly, reduction in processing load can be realized accordingly.

Conclusion

As described above, with the present embodiment, interpersonal distance mean values are calculated not with all points in time during a period to be evaluated but with only a point in time wherein a predetermined condition holds.

Thus, a technique has been employed wherein points in time to be evaluated for obtaining a distance mean value are narrowed down to only a point in time wherein a certain condition holds, whereby an interpersonal relationship evaluation index can be prevented from depending on time length wherein the partner exists in the neighborhood. Thus, misconception of interpersonal relationship can be prevented from occurrence effectively, such as misconception of interpersonal relationship occurring in the case of employing a technique according to the related art with time length wherein the partner exists in the neighborhood as an index, and accordingly, interpersonal relationship can be evaluated in a more accurate manner.

Also, as described above, points in time to be evaluated for obtaining a distance mean value are narrowed down to only a point in time wherein a certain condition holds, whereby a mean value of distance between the respective users can be obtained in a state wherein a predetermined condition is satisfied. Thus, interpersonal relationship can be evaluated based on the behavior of each user (proximity tendency or alienation tendency) in a situation wherein a certain condition is satisfied.

For example, as the above-mentioned predetermined condition, if a condition is set wherein the state of the respective users to be evaluated are a state wherein particular relationship (e.g., friends or lovers) is estimated, such as previously exemplified "horizontal array", "facing", "backside", or the like, interpersonal relationship can be evaluated from the behavior of each user in a situation wherein such particular relationship is estimated.

Alternatively, points in time to be evaluated for obtaining a distance mean value can also be narrowed down to a point in time wherein the partner exists within a target range such as shown in Fig. 5. Thus, calculation of evaluation values with a state wherein existence of the partner is not felt as a target, i.e., with a state wherein no influence is applied to interpersonal relationship as a target can be prevented, and accordingly, interpersonal relationship can be evaluated in a more accurate manner.

Alternatively, points in time to be evaluated for obtaining a distance mean value can also be narrowed down to only a point in time wherein the behavior of the self and the behavior of the partner are synchronized. Thus, calculation of evaluation values with a state to be prevented from being a target to be evaluated as a target, such as a state passing through around a person who simply rests, can be prevented, and accordingly, interpersonal relationship can be evaluated in a more accurate manner.

Thus, according to the technique of the present embodiment wherein points in time to be evaluated for calculating a distance mean value are narrowed down, more accurate interpersonal relationship evaluation values can be obtained.

Second Embodiment

Next, a second embodiment will be described. The second embodiment is an embodiment wherein in addition to various conditions set in the first embodiment, further a condition based on living body information is set.

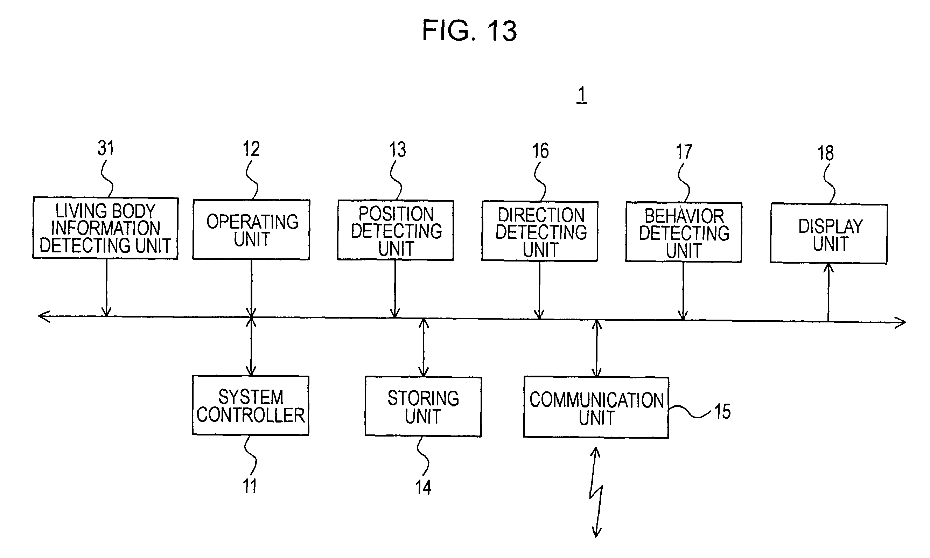

Fig. 13 is a block diagram illustrating the internal configuration example of a terminal device 1 according to the second embodiment. Note that in this diagram, the same components as those described in Fig. 2 are denoted with the same reference numerals, and description thereof will be omitted.

Also, with the second embodiment, the internal configuration of the server device 2 is the same as that described in the first embodiment, so description in the drawing will be omitted.

The terminal device 1 shown in Fig. 13 differs from the terminal device 1 according to the first embodiment in that a living body information detecting unit 31 is added thereto. The living body detecting unit 31 is implemented in the terminal device 1 carried by a user, and includes a portion for sensing living information, for example, such as heartbeat, pulse, or the like, which detects the living information of the user as a numeric value. In this case, the living body information detecting unit 31 is configured so as to detect the heartbeat of the user.

With the second embodiment, a condition based on the above-mentioned heartbeat information is set as a condition for narrowing down points in time to be evaluated fro calculating a distance mean value.

For example, when the respective persons are talking and the conversation is building up, the number of heartbeats (pulse) of both increase. With the second embodiment, this point is utilized, a state wherein the number of heartbeats is synchronized between the user X serving as the self and the user A serving as the partner for each point in time is set as a condition. That is to say, a state wherein the living body state of each user is synchronized is also added to the conditions at the time of setting a point in time to be evaluated for calculating a distance mean value.

Specifically, determination regarding whether or not the condition in this case holds is made by determining regarding whether or not the values of the number of heartbeats at the point in time n of the user X, and the number of heartbeats at the point in time n of the user A are greater than a value equivalent to 150% of the value of heartbeats Hrlx at the time of the same complete rest. A state wherein the conversation of the users X and A is building up can be set to a target to be evaluated for calculating a distance mean value by such a condition setting.

Note that in this case as well, a condition for narrowing down a state serving as a target to be evaluated for calculating a distance mean value is set, whereby a state to be prevented from being a target to be evaluated can be prevented from being included in targets to be evaluated for calculating an evaluation value. That is to say, this point enables calculation of evaluation values in a more accurate manner.

Note that description will be made for confirmation wherein with the terminal device 1 according to the second embodiment, the system controller 11 also transmits the information of the number of heartbeats detected at each point in time at the living body information detecting unit 31 to the server device 2.

Subsequently, with the server device 2, the control unit 21 records the information of the number of heartbeats thus transmitted from the terminal device 1 side in the storing unit 23 in a manner correlated with information at each point in time, thereby accumulating this as the accumulation information for distance calculation 23a. Specifically, the accumulation information for distance calculation 23a in the case of the second embodiment is correlated with an UID, point-in-time information, position information, direction information, acceleration information, and number-of-heartbeats information.

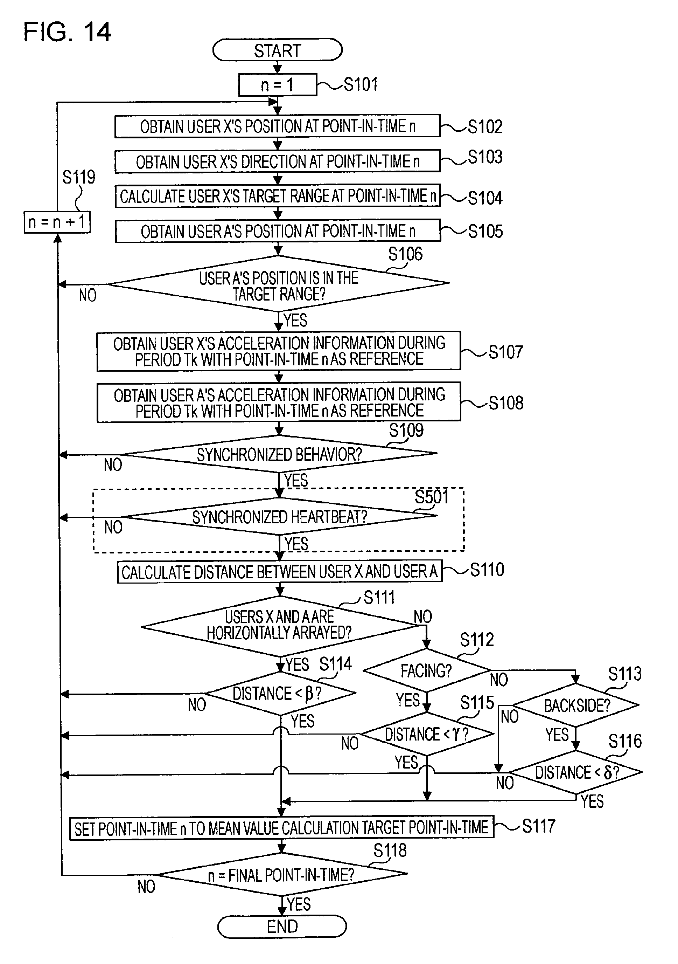

The flowchart shown in Fig. 14 illustrates processing operation to be executed correspondingly in particular at the time of setting a point in time to be evaluated for calculating a distance mean value as processing operation to be executed for realizing operation as the second embodiment described above.

Note that the processing operation shown in Fig. 14 is processing operation which the control unit 21 of the server device 2 according to the second embodiment executes based on the above-mentioned program stored in the ROM.

As can be understood from comparison between the processing operation shown in Fig. 14 and the processing operation shown in Fig. 10, processing for setting a point in time to be evaluated for calculation in the case of the second embodiment is the same setting processing in the case of the first embodiment into which processing for determining whether or not a condition holds in step S501 in the drawing is inserted.

Specifically, the step S501 thereof is executed in a case wherein a positive result has been obtained in step S109 during the series of processing operation shown in Fig. 10. In this step S501, the number-of-heartbeats information of the users X and A at the point in time n, accumulated as the accumulation information for distance calculation, is obtained, and determination is made regarding whether or not these values are greater than 150% of the value of the number of heartbeats Hrlx at the time of predetermined complete rest.

In a case wherein determination is made in step S501 that at least one of the values of the number of heartbeats of the users X and A is not greater than 150% of the value of the number of heartbeats Hrlx at the time of the above-mentioned complete rest, and the heartbeats are not synchronized, and accordingly, a negative result is obtained, in step S119 the value of the identification value n is incremented, and then the processing returns to step S102.

On the other hand, in a case wherein the values of the number of heartbeats of the users X and A are both greater than 150% of the value of the number of heartbeats Hrlx at the time of the above-mentioned complete rest, and accordingly, a positive result is obtained, the processing proceeds to step S110, where distance between the users X and A is calculated.

Such processing is performed, thereby adding a condition wherein a living body state is synchronized as a condition for setting points in time to be evaluated for calculating a mean value.

Modification

Description has been made regarding the embodiments of the present invention so far, but the present invention is not restricted to the specific examples described so far.

For example, the interpersonal relationship evaluation technique of the present invention is not restricted to the case of evaluating interpersonal relationship in the actual world, and can be applied to usage for evaluating interpersonal relationship regarding the respective users in a virtual world over a network, for example, such as "second life" or the like.

In this case, a point wherein system components are multiple terminal devices, and a server device connected to the respective terminal devices through a network so as to perform communication, and a point wherein the respective terminal devices and respective users have one-on-one relationship are unchanged. However, in this case, the position and direction of each user within a virtual world have to be detected, so a technique for detecting a phenomenon or event actually occurring, such as a position detecting unit, direction sensor, or the like, can be eliminated. In this case, the server device manages the placement position and direction to be turned to of a character (icon or the like) representing each user in a virtual world, so such information can be stored for each user and for each point in time successively as the accumulation information for distance calculation 23a. A request from the terminal device 1 side, and calculation and transfer processing of interpersonal relationship evaluation values corresponding thereto are similar to those shown in Figs. 10 through 12, or Fig. 14.

Note that the behavior and living body state (emotion) of each user in a virtual world are also information which the server device side can manage according to progress of an event. Accordingly, in a case wherein a condition based on these behavior and living body state is added as a condition at the time of setting a point in time to be evaluated for calculating a distance mean value, information according to the behavior and living body state thus managed can be accumulated as the accumulation information for distance calculation 23a with the server device side.

Also, with the above description, a case wherein calculated interpersonal relationship evaluation values are employed only for presentation of interpersonal relationship has been exemplified so far, but the interpersonal relationship evaluation values can be employed for other uses.

For example, the interpersonal relationship evaluation values can also be applied to a use such that the setting of the ringtone of a cell phone is automatically changed. In this case, the terminal device 1 side manages the correlation between each user serving as the partner (e.g., the phone number of each user) and ringtone. The terminal device 1 in this case performs a transfer request for interpersonal relationship evaluation values as to each of the partners to the server device 2 in a certain cycle, and updates the correlation between each user and ringtone based on the interpersonal relationship evaluation values as to each of the partners transferred according thereto.

Also, with the above description, the case wherein calculation of interpersonal relationship evaluation values is performed with the server device 2 side has been exemplified, but this may be performed with the terminal device 1 side. Thus, in the case of calculation of interpersonal relationship evaluation values being performed with the terminal device 1 side, the following two patterns can be conceived.

Pattern 1

Step 1: The terminal device 1 requests accumulation information used for calculation to the server device 2 side according to the interpersonal relationship display mode specified by operations, and obtains this.

Step 2: The terminal device 1 performs processing operation shown in Fig. 10, or Figs. 14, 11, and 12 to calculate interpersonal relationship evaluation values, and performs interpersonal relationship display according to the specified display mode based on the calculated interpersonal relationship evaluation values.

Pattern 2

Step 1: The server device 2 executes the processing operation shown in Fig. 10 or Fig. 14, determines a point in time to be evaluated for calculating a distance mean value, and performs calculation of interpersonal distance between users at each point in time to be evaluated.

Step 2: The terminal device 1 performs a transfer request for interpersonal distance information at all points in time to be evaluated regarding respective users used for calculation to the server device 2, and obtains such information. The terminal device 1 executes the processing operation shown in Figs. 11 and 12 based on the obtained interpersonal distance information at all points in time, thereby calculating an interpersonal relationship evaluation value regarding each user, and performing interpersonal relationship display based on the calculated interpersonal relationship evaluation value information.

Also, with the above description, a case wherein various types of information such as a position successively obtained from the terminal device 1 side is accumulated in a correlated manner with the point-in-time information measured on the server device 2 side has been exemplified, but an arrangement may be made wherein the point-in-time information measured on the terminal device 1 side is transferred to the server device 2 side in a correlated manner with the above-mentioned various types of information.

Also, with the above description, description has been made assuming that the calculated interpersonal distance mean values are normalized, but as can be understood from the previous description, normalization is performed particularly at the time of bidirectional interpersonal relationship evaluation results regarding multiple users being requested such as shown in Figs. 9C and 9D to absorb difference in interpersonal distance scales due to the personality of each person or the like, so in a case wherein unidirectional interpersonal relationship evaluation is performed, for example, such as shown in Figs. 9A and 9B, normalization is dispensable.

When this is taken into consideration, and an arrangement may be made wherein switching of calculation processing is performed such that up to normalization is performed at the time of bidirectional evaluation results being requested, but normalization is not performed at the time of unidirectional evaluation results being requested. Thus, normalizing processing which is not indispensable can be prevented from being performed at the time of only unidirectional evaluation results being requested, and reduction in processing load can be realized.

Also, the normalization technique is not restricted to the technique previously exemplified, and for example, the following technique can be employed.

For example, let us consider that the self is a user X, respective partners are users A, B, C, and so on through Z, and normalization is performed regarding interpersonal distance evaluation values $D_{XA}$, $D_{XB}$, $D_{XC}$, and so on through

$D_{XZ}$ of the self X to the respective partners A, B, C, and so on through Z. First, sum $\varepsilon_X$ of the reciprocal numbers of the interpersonal distance mean values $D_{XA}$, $D_{XB}$, $D_{XC}$, and so on through $D_{XZ}$ is obtained with the following calculation.

$$\varepsilon_X = 1 / (1/D_{XA} + 1/D_{XB} + 1/D_{XC} +, \ldots + 1/D_{XZ})$$

Moreover, the following calculation is performed.

$$\varepsilon^*_{XA} = \varepsilon_X / D_{XA}$$

$$\varepsilon^*_{XB} = \varepsilon_X / D_{XB}$$

$$\varepsilon^*_{XC} = \varepsilon_X / D_{XC}$$

and so on through

$$\varepsilon^*_{XZ} = \varepsilon_X / D_{XZ}$$

The values of $\varepsilon^*_{XA}$, $\varepsilon^*_{XB}$, $\varepsilon^*_{XC}$, and so on through $\varepsilon^*_{XZ}$ obtained with the above-mentioned calculation represent percentages occupied by the respective partners A, B, C, and so on through Z with the interpersonal relationship of the self X.

Thus, normalization can be performed so as to obtain a percentage occupied by each partner with the interpersonal relationship of a user serving as the self. Thus, interpersonal distance mean values can be converted into absolute evaluation values wherein difference in interpersonal distance scales due to the personality of each person or the like is absorbed.

Also, with the above description, a case wherein interpersonal relationship evaluation is performed regarding a predetermined period to be evaluated with at least the current point in time as reference has been exemplified, but an arrangement may be made wherein interpersonal relationship evaluation is performed regarding the past predetermined period excluding the current point in time.

Also, with the above description, regarding whether or not a condition regarding a behavior state holds has been determined by determining whether or not the behavior state is synchronized with the classifications of "resting", "walking", and "running", but for example, such as an "Actigraph", an arrangement may be made wherein classification of "active" and "inactive" is performed according to a result wherein frequencies of 0.01 G or more acceleration with a certain period are accumulated, and determination is made regarding whether or not the behavior state is synchronized with the classification thereof.

Also, with the above description, two types of a target range, behavior, and placement state, or a living body state being combined therewith have been exemplified as a condition to be set at the time of narrowing down points in time to be evaluated for calculating a distance mean value, but such a combination of conditions may be an arbitrary combination as appropriate.

Also, a condition to be set at the time of narrowing down points in time to be evaluated for calculating a distance mean value is not restricted to the conditions exemplified with the above description, so a requested condition can be set as appropriate. As can be understood from the previous description, with the present invention, it is important to set a condition to narrow down points in time to be evaluated for calculating a distance mean value, and thus, interpersonal relationship can be evaluated in a more accurate manner.

Also, in the case of performing display of interpersonal relationship, with regard to expression of interpersonal relationship, another technique other than the techniques exemplified previously may be employed, such as expression with color difference in each user, or a line connecting between users, or the like.

Also, with the above description, only a technique employing a GPS unit has been exemplified as a technique for detecting a position by the terminal device 1, but instead of this, Wi-Fi (Wireless Fidelity) or position information service provided by a cell phone company may be employed.

Also, with the above description, a technique employing a direction sensor has been exemplified as a technique for detecting a direction which a user turns to, in addition to this, a technique may be employed wherein a direction which a user turns to is detected by employing an imaging image captured by a camera turning to the front direction of the terminal device 1 (user). For example, determination is made from image analysis results whether or not a user serving as the partner is projected within the above-mentioned imaging image captured by a camera, whereby determination can be made regarding whether or not a placement state of the self as to the partner is a facing state.

Also, with the above description, only a case has been exemplified wherein a device for detecting information used for formation of accumulation information for distance calculation (position, direction, acceleration, living body information, etc.), and a device for obtaining (receiving) evaluation results are the same device, but there is a case wherein a device for obtaining evaluation results is a separate device which does not include various types of detecting units. A specific example of this is a case wherein a certain user possesses the terminal device 1, and an information processing device capable of network connection such as a personal computer, and in this case, a transfer request specifying a UID is performed form the above-mentioned information processing device to the server device, thereby obtaining evaluation results at the information processing device.

In this case, the above-mentioned information processing device has to execute processing for obtaining particular information (calculation results of interpersonal relationship evaluation values, accumulation information used for calculation of interpersonal relationship evaluation values in the case of the above-mentioned patterns 1 and 2, or interpersonal distance information at all points in time to be evaluated for calculation) from the server device side, such that the processing operation on the terminal device side shown in Fig. 12 is executed at the above-mentioned information processing device, or the like.

It should be understood by those skilled in the art that various modifications, combinations, sub-combinations and alterations may occur depending on design requirements and other factors insofar as they are within the scope of the appended claims or the equivalents thereof.

In so far as the embodiments of the invention described above are implemented, at least in part, using software-controlled data processing apparatus, it will be appreciated that a computer program providing such software control and a transmission, storage or other medium by which such a computer program is provided are envisaged as aspects of the present invention.

The following numbered clauses describe matter that may be combined as indicated with the claims:

Clause 1. The interpersonal relationship evaluation device according to Claim 5 dependent upon claim 4 dependent upon claim 2, wherein, with said evaluation value calculation processing, a mean value of distance between the respective terminal devices is calculated regarding only a point in time wherein determination is made in said range condition determining processing that a condition holds wherein said other terminal device exists within said predetermined range, and determination is made in said behavior condition determining processing that a condition holds wherein the behavior of each terminal device user is synchronized, and determination is made in said living body condition determining processing that a condition holds wherein the living body state of each terminal device user is synchronized.

Clause 2. The interpersonal relationship evaluation system according to Claim 9, wherein said sever-side control means further executes range condition determining processing for determining whether or not a condition holds regarding a point in time to be determined wherein the other terminal device exists within a predetermined range with the position of one of the terminal devices serving as an evaluation value calculation target as reference; and wherein, with said evaluation value calculation processing, a mean value of distance between the respective terminal devices is calculated with a point in time as a target wherein determination is made in said range condition determining processing that a condition holds wherein said other terminal device exists within said predetermined range.

Clause 3. The interpersonal relationship evaluation system according to clause 2, wherein said terminal devices further include
direction detecting means configured to detect a direction which the self device turns to;
and wherein with said terminal-side information transmission control processing, control is performed such that the information of a direction sequentially detected at each point in time by said direction detecting means is transmitted to said server device by said terminal-side communication means together with position information detected by said position detecting means;
and wherein with said range condition determining processing by said server-side control means, determination is made whether or not a condition holds regarding a point in time to be determined as a target wherein said other terminal device exists within a predetermined range which has been set according to the direction of said one terminal device.

Clause 4. The interpersonal relationship evaluation system according to clause 2 or clause 3, wherein said terminal devices further include

acceleration detecting means configured to detect acceleration applied to the self device;

and wherein with said terminal-side information transmission control processing, control is performed such that the information of acceleration sequentially detected at each point in time by said acceleration detecting means is transmitted to said server device by said terminal-side communication means together with position information detected by said position detecting means, and direction information detected by said direction detecting means;

and wherein said server-side control means further execute

behavior condition determining processing for determining based on an acceleration change pattern of one of the terminal devices serving as an evaluation value calculation target, and an acceleration change pattern of the other terminal device whether or not a condition holds regarding a point in time to be determined as a target wherein the behavior of each terminal device user is synchronized;

and wherein, with said evaluation value calculation processing, a mean value of distance between the respective terminal devices is calculated with a point in time as a target wherein determination is made in said range condition determining processing that a condition holds wherein said other terminal device exists within said predetermined range, and determination is made in said behavior condition determining processing that a condition holds wherein the behavior of each terminal device user is synchronized.

Clause 5. The interpersonal relationship evaluation system according to any one of clauses 2 to 4, wherein said terminal devices further include

living body information detecting means configured to detect a user's living body information;

and wherein with said terminal-side information transmission control processing, control is performed such that living body information sequentially detected at each point in time by said living body information detecting means is transmitted to said server device by said terminal-side communication means together with position information detected by said position detecting means, direction information detected by said direction detecting means, and the information of acceleration detected by said acceleration detecting means;

and wherein said server-side control means further execute

living body condition determining processing for determining based on said living body information obtained from one of the terminal devices serving as an evaluation value calculation target, and said living body information obtained from the other terminal device whether or not a condition holds regarding a point in time to be determined as a target wherein the living body state of each terminal device user is synchronized;

and wherein, with said evaluation value calculation processing, a mean value of distance between the respective terminal devices is calculated with a point in time as a target wherein determination is made in said range condition determining processing that a condition holds wherein said other terminal device exists within said predetermined range, and determination is made in said behavior condition determining processing that a condition holds wherein the behavior of each terminal device user is synchronized, and determination is made in said living body condition determining processing that a condition holds wherein the living body state of each terminal device user is synchronized.

Clause 6. The interpersonal relationship evaluation system according to any one of clauses 2 to 5, wherein said server-side control means further execute

placement-state condition determining processing for determining based on the position information of one of the terminal devices serving as an evaluation value calculation target, and the position information of the other terminal device whether or not a condition holds regarding a point in time to be determined as a target wherein the placement state of said one terminal device corresponding to said other terminal device is a predetermined placement state;

and wherein, with said evaluation value calculation processing, a mean value of distance between the respective terminal devices is calculated with a point in time as a target wherein determination is made in said range condition determining processing that a condition holds wherein said other terminal device exists within said predetermined range, and determination is made in said behavior condition determining processing that a condition holds wherein the behavior of each terminal device user is synchronized, and determination is made in said living body condition determining processing that a condition holds wherein the living body state of each terminal device user is synchronized, and determination is made in said placement-state condition determining processing that a condition holds wherein the placement state of said one terminal device is a predetermined placement state.

Clause 7. The interpersonal relationship evaluation system according to clause 5 when dependent upon clause 4 when in turn dependent upon clause 2, wherein, with said evaluation value calculation processing, a mean value of distance between the respective terminal devices is calculated regarding only a point in time wherein determination is made in said range condition determining processing that a condition holds wherein said other terminal device exists within said predetermined range, and determination is made in said behavior condition determining processing that a condition holds wherein the behavior of each terminal device user is synchronized, and determination is made in said living body condition determining processing that a condition holds wherein the living body state of each terminal device user is synchronized.

Clause 8. The interpersonal relationship evaluation system according to claim 9 or any one of clauses 2 to 7, wherein said server-side control means further execute
normalizing processing for normalizing interpersonal distance mean values obtained by dividing each of interpersonal distance mean values to other multiple terminal device users from a certain terminal device user obtained in said evaluation value calculation processing by the total value thereof.

Clause 9. The interpersonal relationship evaluation system according to clause 8, wherein said server-side control means further execute
server-side information transmission control processing for performing control such that the interpersonal distance mean values obtained in said evaluation value calculation processing, or the interpersonal distance mean values normalized in said normalizing processing are transmitted to said terminal devices by said server-side communication means;
and wherein said terminal-side control means further execute
reception control processing for performing control such that the information transmitted in said server-side information transmission control processing is received by said terminal-side communication means.

Clause 10. The interpersonal relationship evaluation system according to Claim 9, wherein said terminal devices further include
display means configured to perform display of information;
wherein said terminal-side control means further execute
display control processing for performing control such that display representing interpersonal relationship is performed upon said display means in a display mode corresponding to said interpersonal distance mean values received in said reception control processing, or said normalized interpersonal distance mean values.

Clause 11. The terminal device according to Claim 10, further comprising:

display means configured to perform display of information;

wherein said control means further execute
display control processing for performing control such that display representing interpersonal relationship is performed upon said display means in a display mode corresponding to said interpersonal relationship evaluation values received in said reception control processing.

Clause 12. The terminal device according to any one of claims 10 or 11 or clause 11, further comprising:

acceleration detecting means configured to detect acceleration applied to the self device;

wherein with said information transmission control processing, control is performed such that the information of acceleration sequentially detected at each point in time by said acceleration detecting means is further transmitted to said server device by said communication means together with position information detected by said position detecting means, and the information of a direction detected by said direction detecting means.

Clause 13. The terminal device according to any one of Claims 10 or 11 or clauses 11 or 12, further comprising:

living body information detecting means configured to detect the living body information of a user;

wherein with said information transmission control processing, control is performed such that living body information sequentially detected at each point in time by said living body information detecting means is further transmitted to said server device by said communication means together with position information detected by said position detecting means, the information of a direction detected by said direction detecting means, and the information of acceleration detected by said acceleration detecting means.

**Claims**

1. An interpersonal relationship evaluation device comprising:

computing means configured to execute
information obtaining processing for obtaining position information obtained over a plurality of points in time with each of two or more terminal devices, and
evaluation value calculation processing for calculating a mean value of distance between the respective terminal devices with a point in time when a predetermined condition holds as a target to obtain said mean value as an interpersonal relationship evaluation value regarding each terminal device user.

2. The interpersonal relationship evaluation device according to Claim 1, wherein said computing means further execute range condition determining processing for determining whether or not a condition holds regarding a point in time to be determined as a target wherein the other terminal device exists within a predetermined range with the position of one of the terminal devices serving as an evaluation value calculation target as reference;
and wherein, with said evaluation value calculation processing, a mean value of distance between the respective terminal devices is calculated with a point in time as a target wherein determination is made in said range condition determining processing that a condition holds wherein said other terminal device exists within said predetermined range.

3. The interpersonal relationship evaluation device according to Claim 2, wherein, with said information obtaining processing, the direction information of the respective terminal devices obtained over a plurality of points in time at each of the two or more terminal devices is obtained together with said position information;
and wherein, with said range condition determining processing, determination is made whether or not a condition holds regarding a point in time to be determined as a target wherein said other terminal device exists within a predetermined range which has been set according to the direction of said one of the terminal devices.

4. The interpersonal relationship evaluation device according to any one of claims 2 or 3, wherein, with said information obtaining processing, acceleration information detected over a plurality of points in time at each of the two or more terminal devices is obtained together with said position information and said direction information;
and wherein said computing means further execute
behavior condition determining processing for determining based on an acceleration change pattern of one of the terminal devices serving as an evaluation value calculation target, and an acceleration change pattern of the other terminal device whether or not a condition holds regarding a point in time to be determined as a target wherein the behavior of each terminal device user is synchronized;
and wherein, with said evaluation value calculation processing, a mean value of distance between the respective terminal devices is calculated with a point in time as a target wherein determination is made in said range condition determining processing that a condition holds wherein said other terminal device exists within said predetermined range, and determination is made in said behavior condition determining processing that a condition holds wherein the behavior of each terminal device user is synchronized.

5. The interpersonal relationship evaluation device according to any one of claims 2 to 4, wherein, with said information obtaining processing, the living body information of each terminal device user detected over a plurality of points in time with each of the two or more terminal devices is obtained together with said position information, said direction information, and said acceleration information;
and wherein said computing means further execute
living body condition determining processing for determining based on said living body information obtained from one of the terminal devices serving as an evaluation value calculation target, and said living body information obtained from the other terminal device whether or not a condition holds regarding a point in time to be determined as a target wherein the living body state of each terminal device user is synchronized;
and wherein, with said evaluation value calculation processing, a mean value of distance between the respective terminal devices is calculated with a point in time as a target wherein determination is made in said range condition determining processing that a condition holds wherein said other terminal device exists within said predetermined range, and determination is made in said behavior condition determining processing that a condition holds wherein the behavior of each terminal device user is synchronized, and determination is made in said living body condition determining processing that a condition holds wherein the living body state of each terminal device user is synchronized.

**6.** The interpersonal relationship evaluation device according to any one of claims 2 to 5, wherein said computing means further execute

placement-state condition determining processing for determining based on the position information of one of the terminal devices serving as an evaluation value calculation target, and the position information of the other terminal device whether or not a condition holds regarding a point in time to be determined as a target wherein the placement state of said one terminal device corresponding to said other terminal device is a predetermined placement state; and wherein, with said evaluation value calculation processing, a mean value of distance between the respective terminal devices is calculated with a point in time as a target wherein determination is made in said range condition determining processing that a condition holds wherein said other terminal device exists within said predetermined range, and determination is made in said behavior condition determining processing that a condition holds wherein the behavior of each terminal device user is synchronized, and determination is made in said living body condition determining processing that a condition holds wherein the living body state of each terminal device user is synchronized, and determination is made in said placement-state condition determining processing that a condition holds wherein the placement state of said one terminal device is a predetermined placement state.

**7.** The interpersonal relationship evaluation device according to any one of the preceding claims, wherein said computing means further execute

normalizing processing for normalizing interpersonal distance mean values obtained by dividing each of interpersonal distance mean values to other multiple terminal device users from a certain terminal device user obtained in said evaluation value calculation processing by the total value thereof.

**8.** An interpersonal relationship evaluation method comprising the steps of:

obtaining position information obtained at each of the two or more terminal devices over a plurality of points in time; and
calculating a mean value of distance between the respective terminal devices with a point in time as a target wherein a predetermined condition holds to obtain the mean value as an interpersonal relationship evaluation value regarding each terminal device user.

**9.** An interpersonal relationship evaluation system comprising:

two or more terminal devices; and
a server device;
wherein said terminal devices include
terminal-side communication means configured to perform data communication with said server device;
position detecting means configured to detect a current position; and
terminal-side control means configured to execute terminal-side information transmission control processing for performing control such that position information detected sequentially at each point in time by said position detecting means is transmitted to said server device by said terminal-side communication means;
and wherein said server device includes
server-side communication means configured to perform data communication with said terminal devices;
recording means configured to perform recording of information as to a predetermined recording medium; and
server-side control means;
and wherein said server-side control means execute
recording control processing for performing control as to said recording means such that in response to information transmitted in said terminal-side information transmission control processing being received by said server-side communication means, the received information is recorded in said recording medium in a manner wherein identification can be performed for each of said terminal devices and for each point in time, and evaluation value calculation processing for calculating a mean value of distance between the respective terminal devices with a point in time as a target wherein a predetermined condition holds based on the information recorded in said recording medium in said recording control processing to obtain the mean value as an interpersonal relationship evaluation value regarding each terminal device user.

**10.** A terminal device employed for an interpersonal relationship evaluation system including said two or more terminal devices and a server device, the terminal device comprising:

communication means configured to perform data communication with said server device;
position detecting means configured to detect a current position; and

control means;

wherein said control means execute

information transmission control processing for performing control such that position information sequentially detected at each point in time by said position detecting means is transmitted to said server device by said terminal-side communication means, and

reception control processing for performing control such that interpersonal relationship evaluation values regarding the respective terminal device users, calculated based on said position information transmitted from said respective terminal devices, and transmitted from said server device are received by said terminal-side communication means.

**11.** The terminal device according to Claim 10, further comprising:

direction detecting means configured to detect a direction which the self device turns to;

wherein with said information transmission control processing, control is performed such that the information of a direction sequentially detected at each point in time by said direction detecting means is transmitted to said server device by said communication means together with position information detected by said position detecting means.

**12.** An interpersonal relationship evaluation method comprising the steps of:

obtaining position information over a plurality of points in time regarding each of two or more users; and

calculating a mean value of distance between the respective users with a point in time as a target wherein a predetermined condition holds to obtain the mean value thereof as an interpersonal relationship evaluation value regarding the respective users.

**13.** An interpersonal relationship evaluation device comprising:

a computing unit configured to execute

information obtaining processing for obtaining position information obtained over a plurality of points in time with each of two or more terminal devices, and

evaluation value calculation processing for calculating a mean value of distance between the respective terminal devices with a point in time when a predetermined condition holds as a target to obtain said mean value as an interpersonal relationship evaluation value regarding each terminal device user.

**14.** An interpersonal relationship evaluation system comprising:

two or more terminal devices; and

a server device;

wherein said terminal devices include

a terminal-side communication unit configured to perform data communication with said server device;

a position detecting unit configured to detect a current position; and

a terminal-side control unit configured to execute terminal-side information transmission control processing for performing control such that position information detected sequentially at each point in time by said position detecting unit is transmitted to said server device by said terminal-side communication unit;

and wherein said server device includes

a server-side communication unit configured to perform data communication with said terminal devices;

a recording unit configured to perform recording of information as to a predetermined recording medium; and

a server-side control unit;

and wherein said server-side control unit executes

recording control processing for performing control as to said recording unit such that in response to information transmitted in said terminal-side information transmission control processing being received by said server-side communication unit, the received information is recorded in said recording medium in a manner wherein identification can be performed for each of said terminal devices and for each point in time, and

evaluation value calculation processing for calculating a mean value of distance between the respective terminal devices with a point in time as a target wherein a predetermined condition holds based on the information recorded in said recording medium in said recording control processing to obtain the mean value as an interpersonal relationship evaluation value regarding each terminal device user.

**15.** A terminal device employed for an interpersonal relationship evaluation system including
said two or more terminal devices, and
a server device,
comprising:

a communication unit configured to perform data communication with said server device;
a position detecting unit configured to detect a current position; and
a control unit;

wherein said control unit executes
information transmission control processing for performing control such that position information sequentially detected at each point in time by said position detecting unit is transmitted to said server device by said terminal-side communication unit, and
reception control processing for performing control such that interpersonal relationship evaluation values regarding the respective terminal device users, calculated based on said position information transmitted from said respective terminal devices, and transmitted from said server device are received by said terminal-side communication unit.

# FIG. 1

# FIG. 2

EP 2 065 721 A2

# FIG. 3

<u>2</u>

POINT-IN-TIME
MEASURING UNIT — 22

CONTROL
UNIT — 21

STORING UNIT — 23

ACCUMULATION
INFORMATION
FOR DISTANCE
CALCULATION — 23a

COMMUNICATION
UNIT — 24

EP 2 065 721 A2

## FIG. 4

| UID | POINT IN TIME | DIRECTION | ACCELERATION |
|-----|------|-----------|--------------|
| A | n | 29. 1 | 0. 5 |
| B | n | 30. 3 | 0. 7 |
| C | n | 270. 0 | 2. 1 |
| . | . | . | . |
| A | n + 1 | 30. 2 | 0. 7 |
| B | n + 1 | 45. 0 | 1. 0 |
| C | n + 1 | 271. 0 | 1. 9 |
| . | . | . | . |

## FIG. 5

FORWARD

$\theta$

BACKWARD

## FIG. 6A

DIRECTION VECTOR

X

DISTANCE VECTOR

A

## FIG. 6B

A

DIRECTION VECTOR

DISTANCE VECTOR

X

## FIG. 6C

A

DISTANCE VECTOR

X

DIRECTION VECTOR

# FIG. 7

DISTANCE BETWEEN
USER X AND USER A

CALCULATE A MEAN VALUE OF
DISTANCE AT ALL POINT-IN-TIME WITH
ONLY A PERIOD WHEREIN CONDITIONS
AGREE AS A TARGET

t
(TIME)

CONDITIONS ARE
CLOSE BUT DISAGREE

CONDITIONS ARE
CLOSE BUT DISAGREE

CONDITIONS
AGREE

CONDITIONS
AGREE

# FIG. 8

SERVER DEVICE

·UID
·POSITION
·DIRECTION
·ACCELERATION

2

1    1    1

. . .

. . .

CALCULATE INTERPERSONAL DISTANCE MEAN VALUES
(INTERPERSONAL RELATIONSHIP EVALUATION VALUES)
AS TO OTHER USERS FOR EACH USER

| A→B | B→A | C→A |
| A→C | B→C | C→B |
| ⋮ | ⋮ | ⋮ |

ABSOLUTE INTERPERSONAL
RELATIONSHIP EVALUATION VALUES WITH
(INTROVERTED/EXTROVERTED)
PERSONALITY OF EACH USER BEING
TAKEN INTO CONSIDERATION

NORMALIZE INTERPERSONAL DISTANCE
MEAN VALUES FOR EACH USER

EP 2 065 721 A2

FIG. 9A

FIG. 9B

FIG. 9C

FIG. 9D

FIG. 10

```
                          ┌──────────────┐
                          │    START     │
                          └──────┬───────┘
                                 ▼
                          ┌──────────────┐
                          │    n = 1     │──S101
                          └──────┬───────┘
                                 ▼
            ┌────────────────────────────────────────────┐
            │ OBTAIN USER X'S POSITION AT POINT-IN-TIME n │──S102
            └────────────────────┬───────────────────────┘
                                 ▼
            ┌────────────────────────────────────────────┐
            │ OBTAIN USER X'S DIRECTION AT POINT-IN-TIME n│──S103
            └────────────────────┬───────────────────────┘
                                 ▼
       ┌──────────────────────────────────────────────────────┐
  S119 │ CALCULATE USER X'S TARGET RANGE AT POINT-IN-TIME n    │──S104
       └──────────────────────┬───────────────────────────────┘
  ┌──────────┐                ▼
  │ n = n + 1│  ┌────────────────────────────────────────────┐
  └──────────┘  │ OBTAIN USER A'S POSITION AT POINT-IN-TIME n │──S105
                └────────────────────┬───────────────────────┘
                                     ▼                    S106
                          ╱──────────────────────╲
                    NO   ╱  USER A'S POSITION IS IN ╲
                ◄───────╱     THE TARGET RANGE?      ╲
                         ╲                          ╱
                          ╲────────────┬───────────╱
                                     YES
                                      ▼
         ┌──────────────────────────────────────────────┐
         │ OBTAIN USER X'S ACCELERATION INFORMATION DURING│──S107
         │ PERIOD Tk WITH POINT-IN-TIME n AS REFERENCE    │
         └──────────────────────┬───────────────────────┘
                                ▼
         ┌──────────────────────────────────────────────┐
         │ OBTAIN USER A'S ACCELERATION INFORMATION DURING│──S108
         │ PERIOD Tk WITH POINT-IN-TIME n AS REFERENCE    │
         └──────────────────────┬───────────────────────┘
                                ▼                    S109
                     ╱──────────────────────╲
               NO   ╱   SYNCHRONIZED BEHAVIOR? ╲
           ◄───────╱                           ╲
                    ╲─────────────┬────────────╱
                                YES
                                 ▼
              ┌───────────────────────────────────────┐
              │ CALCULATE DISTANCE BETWEEN USERS X AND A│──S110
              └──────────────────┬────────────────────┘
```

SET POINT-IN-TIME n TO MEAN VALUE CALCULATION TARGET POINT-IN-TIME ──S117

USERS X AND A ARE HORIZONTALLY ARRAYED? ── S111

FACING? ── S112

BACKSIDE? ── S113

DISTANCE < β? ── S114

DISTANCE < γ? ── S115

DISTANCE < δ? ── S116

n = FINAL POINT-IN-TIME? ── S118

END

35

# FIG. 11

```
                    ┌─────────────┐
                    │    START    │
                    └──────┬──────┘
                           │
                           ▼
```

| | |
|---|---|
| CALCULATE A MEAN VALUE OF DISTANCE AT ALL TARGET POINT-IN-TIME FOR EACH PARTNER (CALCULATION OF $D_{XA}$, $D_{XB}$, $D_{XC}$, ···· $D_{XZ}$) | S201 |

| | |
|---|---|
| CALCULATE TOTAL INTERPERSONAL DISTANCE ($D_X = D_{XA} + D_{XB} + D_{XC}$ ···· $+ D_{XZ}$) | S202 |

NORMALIZE INTERPERSONAL DISTANCE
MEAN VALUES AS TO THE RESPECTIVE PARTNERS

$$
\begin{pmatrix}
D^{*}_{XA} = D_{XA}/D_X \\
D^{*}_{XB} = D_{XB}/D_X \\
D^{*}_{XC} = D_{XC}/D_X \\
\vdots \quad\quad \vdots \\
D^{*}_{XZ} = D_{XZ}/D_X
\end{pmatrix}
$$

S203

```
                    ┌─────────────┐
                    │     END     │
                    └─────────────┘
```

# FIG. 12

TERMINAL DEVICE

START

S301

INTERPERSONAL RELATIONSHIP DISPLAY INSTRUCTIONS FROM SELF TO THE RESPECTIVE PARTNERS?

NO

YES

S302

SPECIFY UID TO PERFORM A TRANSFER REQUEST FOR INTERPERSONAL RELATIONSHIP EVALUATION VALUES FROM SELF TO THE RESPECTIVE PARTNERS

S303

RECEIVED?

NO

YES

S304

DISPLAY PROCESSING BASED ON THE EVALUATION VALUES

END

SERVER DEVICE

START

S401

RECEIVED?

NO

YES

CALCULATION PROCESSING OF INTERPERSONAL RELATIONSHIP EVALUATION VALUES AS TO THE RESPECTIVE PARTNERS FROM SELF SPECIFIED WITH THE UID

S402

TRANSFER CALCULATED INTERPERSONAL RELATIONSHIP EVALUATION VALUES

S403

END

EP 2 065 721 A2

# FIG. 13

EP 2 065 721 A2

EP 2 065 721 A2

## FIG. 14

START

$n = 1$ — S101

OBTAIN USER X'S POSITION AT POINT-IN-TIME n — S102

OBTAIN USER X'S DIRECTION AT POINT-IN-TIME n — S103

CALCULATE USER X'S TARGET RANGE AT POINT-IN-TIME n — S104

OBTAIN USER A'S POSITION AT POINT-IN-TIME n — S105

S119

$n = n + 1$

USER A'S POSITION IS IN THE TARGET RANGE? — S106
NO / YES

OBTAIN USER X'S ACCELERATION INFORMATION DURING PERIOD Tk WITH POINT-IN-TIME n AS REFERENCE — S107

OBTAIN USER A'S ACCELERATION INFORMATION DURING PERIOD Tk WITH POINT-IN-TIME n AS REFERENCE — S108

SYNCHRONIZED BEHAVIOR? — S109
NO / YES

SYNCHRONIZED HEARTBEAT? — S501
NO / YES

CALCULATE DISTANCE BETWEEN USER X AND USER A — S110

USERS X AND A ARE HORIZONTALLY ARRAYED? — S111
YES S114 / NO

DISTANCE < β? — S114
NO / YES

FACING? — S112
YES / NO

BACKSIDE? — S113
YES / NO

DISTANCE < γ? — S115
NO / YES

DISTANCE < δ? — S116
YES / NO

SET POINT-IN-TIME n TO MEAN VALUE CALCULATION TARGET POINT-IN-TIME — S117

$n =$ FINAL POINT-IN-TIME? — S118
NO / YES

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005131748 A
- JP 2005327156 A